# EUROPÄISCHE PATENTSCHRIFT

(11) **EP 3 448 864 B1**
(45) Veröffentlichungstag und Bekanntmachung des Hinweises auf die Patenterteilung: **23.06.2021**
(21) Anmeldenummer: 17720470.8
(22) Anmeldetag: 27.04.2017
(51) Int. Cl.: C07F 7/21, A61K 31/695, G01N 33/52, A61K 47/52, A61K 47/54, A61K 49/00, G01N 33/84

(54) **SILSESQUIOXANE ALS TRANSPORTVERBINDUNG**
SILSESQUIOXANES AS TRANSPORT COMPOUNDS
SILSESQUIOXANES UTILISÉS COMME COMPOSÉS PORTEURS

(30) Priorität: 29.04.2016 DE 102016107968
(43) Veröffentlichungstag der Anmeldung: 06.03.2019
(73) Patentinhaber: Technische Universität Darmstadt, 64289 Darmstadt (DE)
(72) Erfinder: KNAUER, Sascha, 64291 Darmstadt (DE); KOLMAR, Harald, 64367 Mühltal (DE); HÖRNER, Sebastian, 55116 Mainz (DE); UTH, Christina, 64283 Darmstadt (DE); AVRUTINA, Olga, 64285 Darmstadt (DE)
(74) Vertreter: Fuchs Patentanwälte Partnerschaft mbB
(86) Internationale Anmeldenummer: PCT/EP2017/060099
(87) Internationale Veröffentlichungsnummer: WO 2017/186866

(56) Entgegenhaltungen:
- EP-A1- 2 840 087
- WO-A1-2012/144480
- WO-A1-2013/150444
- WO-A1-2016/021402
- WO-A1-2016/209165
- WO-A2-2014/170833
- CN-B- 102 603 786
- JP-A- 2013 032 340
- ZHAOFU FEI ET AL: "Silsesquioxane Chemistry. IX [1] Synthesis and Characterization of the First Amino- and Iminosilsesquioxanes", ZEITSCHRIFT FUR ANORGANISCHE UND ALLGEMEINE CHEMIE., Bd. 628, Nr. 9-10, 2002, Seiten 2109-2112, XP055395617, DE ISSN: 0044-2313, DOI: 10.1002/1521-3749(200209)628:9/10<2109::AI D-ZAAC2109>3.0.CO;2-H
- SEBASTIAN FABRITZ ET AL: "Bioconjugation on cube-octameric silsesquioxanes", ORGANIC & BIOMOLECULAR CHEMISTRY, Bd. 11, Nr. 14, 24. Oktober 2012 (2012-10-24), Seite 2224, XP055395608, GB ISSN: 1477-0520, DOI: 10.1039/c2ob26807h
- Maria Angela Castriciano ET AL: "A new supramolecular polyhedral oligomeric silsesquioxanes (POSS)-porphyrin nanohybrid: synthesis and spectroscopic characterization", Journal of Materials Chemistry C, vol. 1, no. 31, 1 January 2013 (2013-01-01), page 4746, XP55627405, UK ISSN: 2050-7526, DOI: 10.1039/c3tc30532e
- Paola Cardiano ET AL: "POSS-Tetraalkylammonium Salts: A New Class of Ionic Liquids", EUROPEAN JOURNAL OF INORGANIC CHEMISTRY - CHEMISCHE BERICHTE, vol. 2012, no. 34, 1 December 2012 (2012-12-01), pages 5668-5676, XP55627399, DE ISSN: 1434-1948, DOI: 10.1002/ejic.201200874
- Micha Dutkiewicz ET AL: "Functionalization of Polyhedral Oligomeric Silsesquioxane (POSS) via Nucleophilic Substitution", SYNTHESIS, vol. 2009, no. 12, 12 June 2009 (2009-06-12), pages 2019-2024, XP055627394, STUTTGART, DE. ISSN: 0039-7881, DOI: 10.1055/s-0029-1216807
- Takuhiro Ishii ET AL: "Facile Preparation of Ionic Liquid Containing Silsesquioxane Framework", BULLETIN OF THE CHEMICAL SOCIETY OF JAPAN, vol. 87, no. 1, 15 January 2014 (2014-01-15), pages 155-159, XP055627407, JP ISSN: 0009-2673, DOI: 10.1246/bcsj.20130246

## Beschreibung

Die Erfindung betrifft Verbindungen, die geeignet sind, Moleküle über Zellmembranen hinweg zu transportieren. Die Verbindungen dieser Erfindung gehören zu den Silsesquioxanverbindungen mit der allgemeinen Formel [RSiO_{3/2}]ₓ. Bekannt sind Verbindungen mit 6, 8, 10 und 12 Si-Eckpunkten sowie Polymere. Die Strukturen werden häufig als T6, T8, T10 und T12 bezeichnet (T = Tetraeder-Eckpunkte). Die T8-Strukturen sind die am häufigsten untersuchten Strukturen, sie besitzen die Formel [RSiO_{3/2}]₈ oder äquivalent R₈Si₈O₁₂.

### Stand der Technik

Aus dem Stand der Technik sind Transportverbindungen bekannt, die geeignet sind, Moleküle über Zellmembranen hinweg zu transportieren. Es sind vor allem Peptide wie TAT und Penetratin, die im Stand der Technik für solche Zwecke eingesetzt wurden (vgl. Derossit et al., J. Biol. Chem. 1994, 269, 10444-10450; und Ho et al., Cancer Res. 2001, 61, 474-477). Auch die Verwendung von kubischen Silsesquioxanen wurde im Stand der Technik vorgeschlagen (Fabritz et al., Org. Biomol. Chem., 2013, 11, 2224).

Zhaofu Fei et al. offenbaren in "Silsesquioxane Chemistry. IX [1] Synthesis and characterization of the First Amino- and Iminosilsesquioxanes", Z. Anorg. Allg. Chem., Bd. 628, Nr. 9-10, 01. September 2002, S. 2109-2112 die Synthese und Charakterisierung von Amino- und Iminosilsesquioxanen. Dabei werden aus dem *closo*-Silsesquioxan (c-C₆H₁₁)₇Si₈O₁₂Cl in mehreren Schritten die Amino- und Iminosilsesquioxane hergestellt. Darunter sind auch zwei guanidinsubstituierte Octasilsesquioxane mit jeweils sieben Cyclohexylgruppen zu finden. Die offenbarten Verbindungen sollen als Synthesebausteine Verwendung finden. Einen Bezug zu Cargo-Molekülstrukturen weist diese Veröffentlichung nicht auf.

Die WO 2012/144480 A1 offenbart achtfach identisch substituierte Octasilsesquioxane als härtbare Vorstufen von Polymeren. Die dabei verwendeten Silsesquioxan-Anionen sind über eine ionische Verbindung an kationische quartäre Ammoniumgruppen beinhaltende Reste gebunden. Reste mit einer Cargo-Molekülstruktur sind auch hier nicht vorhanden.

Die WO 2014/170833 A2 offenbart Octasilsesquioxane, die mit Seitenketten substituiert sind, die quartäre Ammoniumgruppen und eine chromophore Gruppe tragen. Diese Verbindungen dienen als gefärbte geladene Partikel in elektrophoretischen Vorrichtungen. Die Seitenketten sind dabei über Alkyl- oder Aryl-Dialkylsilyloxy-Gruppen mit dem Octasilsesquioxangerüst verbunden. Auch hier sind Reste mit einer Cargo-Molekülstruktur nicht vorhanden.

Die WO 2013/150444 A1 offenbart ebenfalls in Verbindung mit elektrophoretischen Vorrichtungen Octasilsesquioxane als gefärbte geladene Partikel. Die mit acht Substituenten versehenen Octasilsesquioxane enthalten Sulfonatgruppen an den Seitenketten. Diese bilden jeweils mit einem eine quartäre Ammoniumgruppe enthaltenden Siloxan als Gegenionen ein Salz. In den Seitenketten ist zudem jeweils eine chromophore Gruppe enthalten. Es ist wiederum keine Cargo-Molekülstruktur offenbart, die an den Seitenketten angeordnet wäre.

In dem Übersichtsartikel von Sebastian Fabritz et al.: "Bioconjugation on cube-octameric silsesquioxanes", Org. Biomol. Chem., Bd. 11, Nr. 14, 01. Januar 2013, S. 2224 werden Biokonjugate mit kubischen Octasilsesquioxanen beschrieben. Die Konjugate weisen dabei keine Ammonium- oder Guanidiniumgruppen in ihren Substituenten auf. Es werden ferner diverse Anwendungsmöglichkeiten der POSS-Verbindungen vorgestellt, insbesondere als bimodale Fluoreszenz- und ¹⁹F-NMR-Sonden.

WO 2016/209165 A1 offenbart ein modifiziertes geschichtetes Tonmaterial. Darin ist beispielsweise ein guanidinoethylaminoethyl-funktionalisiertes Octasilsesquioxan enthalten, welches im Übrigen mit sieben weiteren *tert*-Butylgrupen substituiert ist. Dementsprechend weisen diese Verbindungen keinerlei Cargo-Moleküle auf, die transportiert werden sollen. Die Ankopplung der Guanidingruppe an das Gerüst erfolgt hier über eine Ethylaminoethylgruppierung.

In der WO 2016/021402 A1 wird ebenfalls ein organisch-anorganisch geschichtetes Material vom Perovskit-Typ offenbart, in dem ein durch Substitution eines Octasilsesquioxans mit Ammoniumalkylgruppen erzeugtes Octasilsesquioxankation enthalten ist. Die Substitution erfolgt für alle Seitengruppen gleich, um die weiteren Schichten beiderseits über ionische Verbindungen anbinden zu können. Dementsprechend weisen diese Verbindungen keinerlei Cargo-Moleküle auf. Das Material findet Einsatz als Halbleitermaterial, Lumineszenzmaterial und magnetisches Material.

Die EP 2 840 087 A1 offenbart wiederum ein mit Guanidylalkyl-Gruppen modifiziertes Octasilsesquioxan. Dieses dient der Härtung von alkoxysilylgruppenhaltigen Verbindungen für Kleb- und Dichtstoffe. Auch hier finden sich neben den mit Guanidylalkyl-Gruppen modifizierten Seitenketten keinerlei Cargo-Moleküle.

Ferner wird in dem Artikel von M. A. Castriciano et al.: "A new supramolecular polyhedral oligomeric silsesquioxanes (POSS)-porphyrin nanohybrid: synthesis and spectroscopic characterization", Journal of Material Chemistry C, Bd. 1, Nr. 31, 1. Januar 2013, S. 4746 ein Salz aus einem Porphyrinderivat und einem Silsesquioxan mit einer Trimethylpropylammonium-Substitution offenbart. Die restlichen sieben Substituenten enthalten aber keine Cargo-Molekülstruktur. Bei den beschriebenen Verbindungen handelt es sich um ionische Flüssigkeiten.

Das gleiche Trimethylpropylammonium hepta(isooctyl)octasilsesquioxan-Kation als Salzkomponente von ionischen Flüssigkeiten beschreibt auch der Artikel von P. Cardiano et al.: "POSS-Tetraalkylammonium Salts - A New Class of lonic Liquids", European Journal of Inorganic Chemistry - Chemische Berichte, Bd. 2012, Nr. 34, 1. Dezember 2012, S. 5668-5676. Dieses wird mit sechs verschiedenen Anionen kombiniert und deren Eigenschaften bestimmt. Neben dem Rest mit der quartären Ammoniumgruppe ist kein Rest mit einem Cargo-Molekül an dem Grundgerüst verankert, sondern sieben identische Alkylreste. Bezüglich eines Wirkstoff- und Gentransports wird erwähnt, dass die offenbarten Verbindungen als Baustein für amphiphile Polymere nutzbar sind, welche dann hierfür eingesetzt werden können.

M. Dutkiewicz et al.: "Functionalization of Polyhedral Oligomeric Silsesquioxanes (POSS) via Nucleophilic Substitution", Synthesis, Bd. 2009, Nr. 12, 12. Juni 2009, S. 2019-2024 beschreibt fünf verschiedene funktionalisierte Silsesquioxane. Sie sind sämtlich mit acht gleichen Substituenten ausgestattet und enthalten daher keine Cargo-Molekülstruktur.Der Artikel von T. Ishii et al.: "Facile Preparation of lonic Liquid Containing Silsesquioxane Framework", Bulletin of the Chemical Society of Japan, Bd. 87, Nr. 1, 15. Januar 2014, S. 155-159 betrifft ebenfalls ionische Flüssigkeiten und offenbart das gleiche Trimethylpropylammonium hepta(isooctyl)octasilsesquioxan-Kation wie die Artikel von M. A. Castriciano et al. und P. Cardiano et al.

Die CN 102 603 786 B offenbart eine Reihe von ammoniumsubstituierten Silsesquioxanen, bei denen Alkyleneinheiten die Ammoniumgruppen mit dem Gerüst verbinden, wobei zwischen diesen noch eine CH₂-S-Gruppe eingefügt ist. Es liegt also eine Struktureinheit "Silsesquioxan-CH₂-S-X-A" vor. Auch die Silsesquioxane dieser Veröffentlichung sind achtfach identisch substituiert und tragen kein Cargo-Molekül.

Die JP 2013 032340 A betrifft schließlich ein quartäres Ammoniumsalz und einen daraus hergestellten Oxidationskatalysator zur Produktion von Epoxyderivaten. An einem T8 Gerüst sind dabei sieben (Fluoro-)Alkylreste R¹ angebracht sowie über eine C₁-C₂₀ Alkylengruppe Z eine Ammoniumgruppe. Die einzelnen Methyleneinheiten in R¹ und Z können dabei auch durch Phenylen- oder Oxyeinheiten ersetzt werden. Auch hier liegt also bei den sieben anderen Resten keine Cargo-Molekülstruktur vor.

Insbesondere die Peptide aus dem Stand der Technik haben den Nachteil, dass sie eine sehr kurze Halbwertszeit im physiologischen Milieu aufweisen. Sie sind somit für die Verabreichung von Arzneistoffen nicht geeignet. Andere Verbindungen wie die bekannten Silsesquioxane werden nur sehr langsam in die Zellen aufgenommen, so dass nur schwer die erforderlichen Wirkstoffspiegel erreicht werden. Die Transportverbindungen sollten auch in Anwesenheit von Serum gut in Zellen aufgenommen werden. Es besteht ein Bedarf an Transportverbindungen, die bereits bei sehr geringen extrazellulären Konzentrationen in ausreichendem Maße in Zellen aufgenommen werden. Darüber hinaus wäre es wünschenswert, eine Transportverbindung zu haben, welche Zellmembranen verschiedener Spezies überwinden kann.

Diese Anforderungen werden von den Verbindungen dieser Erfindung erfüllt.

### Beschreibung der Erfindung

Die vorliegende Erfindung betrifft substituierte Silsesquioxanverbindung mit der allgemeinen Formel [RSiO_{3/2}]ₓ. Bekannt sind 6, 8, 10 und 12 Si-Eckpunkte sowie Polymere. Die Strukturen werden häufig als T6, T8, T10 und T12 bezeichnet (T = Tetraeder-Eckpunkte). Die T8-Strukturen sind die am häufigsten untersuchten Strukturen, sie besitzen die Formel [RSiO_{3/2}]8 oder äquivalent R₈Si₈O₁₂. Diese Erfindung betrifft sowohl die T8- als auch die T6-, T10- und T12-Strukturen. Die erfindungsgemäßen Verbindungen haben folgende T6- bis T12-Grundstrukturen:

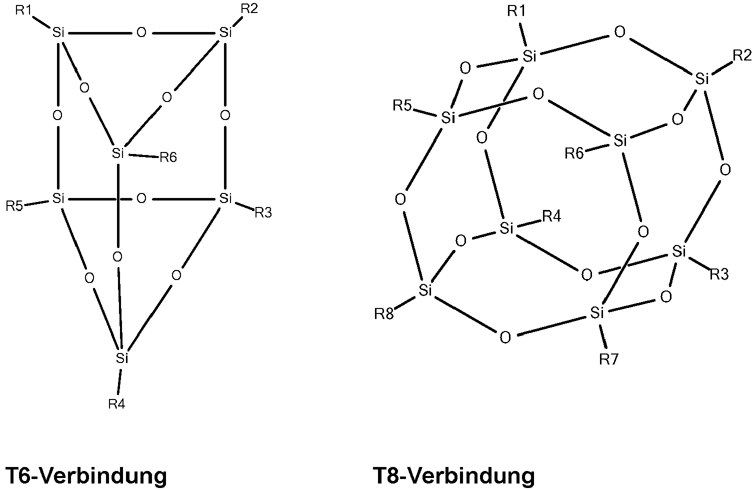

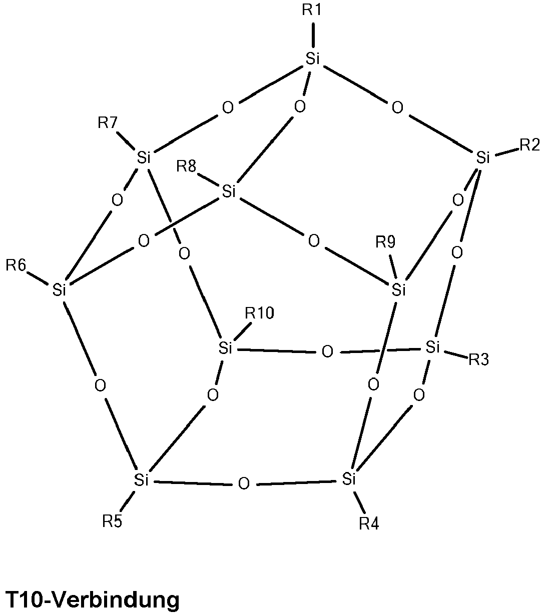

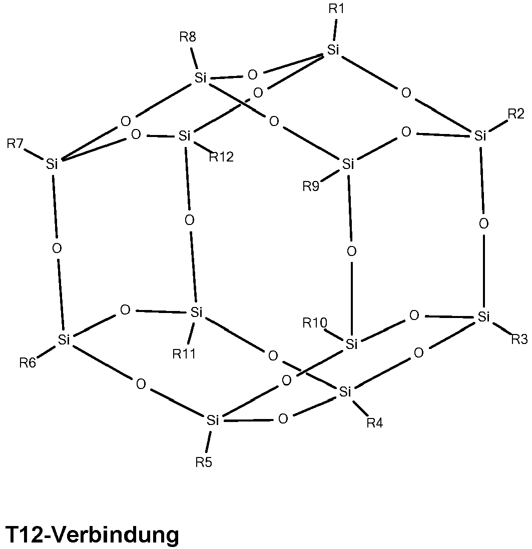

Bei der T8-Verbindung gibt es acht Reste R1 bis R8. T6-, T10- und T12-Verbindungen haben entsprechende sechs (R1 bis R6), zehn (R1 bis R10) bzw. zwölf (R1 bis R12) Reste.

In der erfindungsgemäßen Verbindung ist wenigstens einer aus R1 bis R6 (T6), R1 bis R8 (T8), R1 bis R10 (T10) bzw. R1 bis R12 (T12) ein Rest, welcher eine oder mehrere Guanidingruppen und/oder quartäre Ammoniumgruppen aufweist. Es hat sich heraus gestellt, dass diese beiden funktionellen Gruppen erheblich zur Membrangängigkeit der Verbindungen beitragen. Insbesondere hat es sich gezeigt, dass diese funktionellen Gruppen dazu führen, dass die Verbindungen in großem Ausmaß Zellmembranen überwinden können und dabei sogar Cargo-Moleküle mitnehmen können. Es wird angenommen, dass dies mit der starken Basizität und damit meist positiver Ladung der Guanidingruppe im physiologischen Milieu beziehungsweise der permanenten positiven Ladung der quartären Ammoniumgruppe zusammen hängt.

Die Verbindungen dieser Erfindung zeichnen sich durch eine sehr schnelle Aufnahme in Zellen auch bei sehr geringen Konzentrationen aus. Die Aufnahme in die Zellen wird - anders als bei anderen Verbindungen - nicht durch die Anwesenheit von Serum verringert, was besonders für therapeutische Anwendungen von Bedeutung ist. Die Verbindungen zeichnen sich ferner durch ihre geringe Toxizität aus und die sehr nützliche Eigenschaft, dass sie sich innerhalb relativ kurzer Zeit komplett zersetzen, so dass keine Akkumulation zu befürchten ist. Darüber hinaus können diese Verbindungen in Bakterien, Hefen und Archaeen eindringen.

Erfindungsgemäß umfasst wenigstens einer der Reste aus R1 bis R6 bei T6, R1 bis R8 bei T8, R1 bis R10 bei T10 oder R1 bis R12 bei T12 wenigstens eine Cargo-Molekülstruktur. Die Cargo-Molekülstruktur enthält das Molekül, welches mit Hilfe der erfindungsgemäßen Verbindungen über Zellmembranen transportiert werden soll, vorzugsweise kovalent gebunden. Da die erfindungsgemäßen Verbindungen Cargo-Moleküle transportieren können, ist es erfindungsgemäß, an den Verbindungen dieser Erfindung Cargo-Molekülstrukturen vorzusehen, die eine Funktion im gewünschten Umfeld erfüllen. Diese Cargo-Moleküle sind an die Silsesquioxanstruktur der erfindungsgemäßen Verbindung direkt oder indirekt, vorzugsweise kovalent, gebunden. Die Cargo-Molekülstruktur ist vorzugsweise ausgewählt aus pharmazeutischen Wirkstoffen, Markerverbindungen und Targeting-Strukturen.

Geeignete pharmazeutische Wirkstoffe sind solche, die im menschlichen oder tierischen Körper eine pharmakodynamische Wirkung entfalten. Besonders bevorzugt sind dies Wirkstoffe, die innerhalb von Zellen ihre Wirkung entfalten. Bevorzugte Beispiele sind Chemotherapeutika, Antibiotika und antivirale Arzneistoffe. Ein bevorzugtes Beispiel ist Doxorubicin.

Geeignete Markerverbindungen sind Verbindungen, die innerhalb von Zellen ein messbares Signal erzeugen können. Dies kann ein Fluoreszenzsignal, Phosphoreszenzsignal, radioaktives Signal, Immunsignal oder ähnliches sein. Ein Beispiel für eine Markerverbindung ist das hierin verwendete TAMRA (Tetramethylrhodamin).

Targeting-Strukturen sind Strukturelemente, die geeignet sind, die Verbindungen dieser Erfindung an einem gewünschten Wirkort anzureichern. Typische Targeting-Strukturen sind Antikörper.

Vorzugsweise enthält nur einer oder zwei der Reste aus R1 bis R6 bei T6, R1 bis R8 bei T8, R1 bis R10 bei T10 oder R1 bis R12 bei T12 wenigstens eine, insbesondere genau eine, Cargo-Molekülstruktur. Bevorzugt sind Ausführungsformen, in denen die erfindungsgemäßen Verbindungen nur eine Cargo-Molekülstruktur enthalten, die insbesondere ein pharmazeutischer Wirkstoff oder eine Markerverbindung sein kann. In Fällen mit zwei Cargo-Molekülstrukturen können diese gleich oder unterschiedlich sein. Vorzugsweise sind sie unterschiedlich und eine ist ein pharmazeutischer Wirkstoff oder eine Markerverbindung und die andere ist eine Targeting-Struktur, z.B. ein Antikörper.

Vorzugweise weisen bei T6 wenigstens zwei, mehr bevorzugt wenigstens vier (insbesondere genau vier) oder wenigstens fünf (insbesondere genau fünf) der Reste R1 bis R6 (T6) eine oder mehrere Guanidingruppen und/oder quartäre Ammoniumgruppen auf. Vorzugweise weisen bei T8 wenigstens vier, mehr bevorzugt wenigstens 6 (insbesondere genau 6) oder wenigstens 7 (insbesondere genau 7) der Reste R1 bis R8 eine oder mehrere Guanidingruppen und/oder quartäre Ammoniumgruppen auf. Vorzugweise weisen bei T10 wenigstens sechs, mehr bevorzugt wenigstens acht (insbesondere genau acht) oder wenigstens neun (insbesondere genau neun) der Reste R1 bis R10 eine oder mehrere Guanidingruppen und/oder quartäre Ammoniumgruppen auf. Vorzugweise weisen bei T12 wenigstens acht, mehr bevorzugt wenigstens zehn (insbesondere genau zehn) oder wenigstens elf (insbesondere genau elf) der Reste R1 bis R12 eine oder mehrere Guanidingruppen und/oder quartäre Ammoniumgruppen auf. Es hat sich gezeigt, dass eine größere Zahl an Guanidin- bzw. Ammoniumgruppen die Membrangängigkeit der Verbindungen erhöht. Die Silsesquioxan-Grundstruktur der erfindungsgemäßen Verbindungen kann bei T6 bis zu 6 Substituenten R1 bis R6, bei T8 acht Substituenten R1 bis R8, bei T10 zehn Substituenten R1 bis R10 und bei T12 zwölf Substituenten R1 bis R12 tragen. Wenigstens eine Position trägt einen Rest mit einer Cargo-Molekülstruktur. Es ist dabei nicht ausgeschlossen, dass die Cargo-Molekülstruktur selbst auch eine quartäre Ammoniumverbindung oder eine Guanidingruppe aufweist. Insbesondere werden Antikörper üblicherweise auch Arginin als Aminosäure und somit Guanidingruppen enthalten. Auch weisen einige Arzneistoffe quartäre Ammoniumgruppen auf.

In erfindungsgemäßen Ausführungsformen besteht der Rest (bzw. die Reste), der eine oder mehrere Guanidingruppen und/oder quartäre Ammoniumgruppen enthält, aus der Strukturgruppe X-A,
wobei A ausgewählt ist aus Guanidingruppe und quartärer Ammoniumgruppe, und
wobei X substituiert oder unsubstituiert sein kann und ausgewählt ist aus verzweigtem oder unverzweigtem, zyklischem oder nicht zyklischem, gesättigtem oder ungesättigtem Alkylen und der Gruppe X₁-L-X₂,
wobei X₁ und X₂ jeweils unabhängig voneinander substituiert oder unsubstituiert sein können und aus verzweigtem oder unverzweigtem, zyklischem oder nicht zyklischem, gesättigtem oder ungesättigtem Alkylen ausgewählt sind,
wobei L in der Gruppe X₁-L-X₂ eine Linkergruppe ist, die ausgewählt ist aus Carbonsäureamidgruppe, insbesondere Aminocarbonylgruppe (-HN-CO-) oder Carbonylaminogruppe (-CO-NH-); Carbonsäureestergruppe, insbesondere Oxycarbonyl (-O-CO-) oder Carbonyloxygruppe (-CO-O-), einschließlich der entsprechenden Thioester, Selenoester oder Phosphorester; Ethergruppe; Thioethergruppe; Imidgruppe; Imingruppe; Oxime; Sulfone einschließlich Sulfonamingruppen; Peroxidgruppen; Ketongruppen; Hydrazingruppen; Hydrazongruppen; Carbonsäurehydrazidgruppen und Disulfidbrücken. Bevorzugt ist L ausgewählt aus Carbonsäureamid- und Estergruppe. Besonders bevorzugt sind Verbindungen der genannten Art, wobei X, X₁ und/oder X₂ gesättigtes, nicht-zyklisches C₁- bis C₁₀-Alkylen, insbesondere C₁- bis C₈-Alkylen, bevorzugt C₂-bis C₅-Alkylen und besonders bevorzugt C₃-Alkylen sind. In bevorzugten Ausführungsformen ist L eine Carbonsäureamidgruppe.

Die quartäre Ammoniumgruppe ist vorzugsweise eine Gruppe der Formel -N⁺RₓR_{y}R_{z}, wobei Rₓ, R_{y} und R_{z} unabhängig voneinander ausgewählt sind aus gesättigtem, nicht-zyklischem C₁- bis C₅-Alkyl, insbesondere C₁- bis C₃-Alkyl, bevorzugt ist Methyl. Die quartäre Ammoniumgruppe ist permanent positiv geladen, was eine bessere Wasserlöslichkeit im Vergleich zu Aminogruppen zur Folge hat. Darüber hinaus hat es sich gezeigt, dass quartäre Ammoniumgruppen die Durchquerung von Zellmembranen erleichtern.

Es ist auch eine Verbindung erfindungsgemäß, in der alle Reste aus R1 bis R6 bei T6, R1 bis R8 bei T8, R1 bis R10 bei T10 oder R1 bis R12 bei T12, die eine oder mehrere Guanidingruppen und/oder quartäre Ammoniumgruppen aufweisen, die gleiche chemische Konstitution haben.

Erfindungsgemäß ist auch eine Verbindung wie hierin beschrieben, wobei diejenigen Reste R1 bis R6 bei T6, R1 bis R8 bei T8, R1 bis R10 bei T10 oder R1 bis R12 bei T12, die wenigstens eine Cargo-Molekülstruktur enthalten, eine Strukturgruppe Y-Z aufweisen oder aus der Strukturgruppe Y-Z bestehen, wobei
Y substituiert oder unsubstituiert sein kann und aus verzweigtem oder unverzweigtem, zyklischem oder nicht zyklischem, gesättigtem oder ungesättigtem Alkylen ausgewählt ist, und wobei
Z die Cargo-Molekülstruktur enthält oder aus ihr besteht, wobei Z über eine funktionelle Gruppe direkt oder indirekt an Y gebunden ist, wobei die funktionelle Gruppe ausgewählt ist aus Carbonsäureamidgruppe, insbesondere Aminocarbonylgruppe (-HN-CO-) oder Carbonylaminogruppe (-CO-NH-); Carbonsäureestergruppe, insbesondere Oxycarbonyl (-O-CO-) oder Carbonyloxygruppe (-CO-O-), einschließlich der entsprechenden Thioester, Selenoester oder Phosphorester; Ethergruppe; Thioethergruppe; Imidgruppe; Imingruppe; Oxime; Sulfone einschließlich Sulfonamingruppen; Peroxidgruppen; Ketongruppen; Hydrazingruppen; Hydrazongruppen; Carbonsäurehydrazidgruppen und Disulfidbrücken. Bevorzugt ist die funktionelle Gruppe ausgewählt aus Carbonsäureamid- und Estergruppe. Dem Fachmann sind zahlreiche Möglichkeiten bekannt, um Cargo-Moleküle an die erfindungsgemäße Grundstruktur zu binden. Die genannten Möglichkeiten sind daher nur beispielhaft und sie sollen den Erfindungsgegenstand nicht einschränken.

In einer Ausführungsform weist die Verbindung fünf (im Fall von T6), sieben (im Fall von T8), neun (im Fall von T10) oder elf (im Fall von T12) Reste mit der Strukturgruppe X-A auf und einen Rest mit einer Strukturgruppe Y-Z. In einer anderen Ausführungsform weist die Verbindung vier (im Fall von T6), sechs (im Fall von T8), acht (im Fall von T10) oder zehn (im Fall von T12) Reste mit der Strukturgruppe X-A auf und zwei Reste mit einer Strukturgruppe Y-Z.

In einer bevorzugten Verbindung weist Z folgende Cysteinstruktur auf, welche direkt oder indirekt mit der Cargo-Molekülstruktur (hier mit Q gekennzeichnet) verbunden ist:

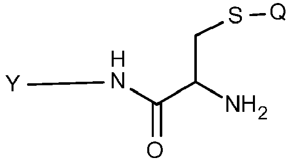

Wenn es hierin heißt, ein Strukturelement der Verbindung sei direkt oder indirekt mit einem anderen Strukturelement verbunden, so ist im Falle einer direkten Verbindung gemeint, dass sich zwischen den verbundenen Gruppen keine weiteren Strukturelemente befinden, sondern die Gruppen unmittelbar kovalent gebunden vorliegen. Bei einer indirekten Verbindung kann zwischen den genannten Gruppen eine weitere Verbindungsstruktur vorgesehen sein.

Erfindungsgemäß sind auch Verbindungen wie oben beschrieben zur Verwendung in einem therapeutischen oder diagnostischen Verfahren. Beispielsweise können die Verbindungen dieser Erfindung in einem Verfahren zur Verabreichung eines pharmazeutischen Wirkstoffes verwendet werden, wobei der pharmazeutische Wirkstoff als Cargo-Molekülstruktur an die Silsesquioxanverbindung gebunden ist. Bevorzugte Beispiele für Diagnoseverfahren sind bildgebende Verfahren, bei denen die Cargo-Molekülstruktur eine Markerverbindung ist. Die Verbindungen werden vorzugsweise parenteral verabreicht.

Erfindungsgemäß ist auch eine Verbindung gemäß dieser Erfindung zur Verwendung in einem Verfahren, bei dem Moleküle über Zellmembranen transportiert werden. Dabei sind ganz unterschiedliche Anwendungen vorstellbar. Beispielsweise können Cargo-Moleküle zu Forschungszwecken über Zellmembranen hinweg transportiert werden.

Ebenfalls hierin beschrieben wird eine Zusammensetzung, z.B. ein pharmazeutisches Präparat, welches die Verbindungen dieser Erfindung umfasst. Die Zusammensetzung ist insbesondere wässrig und hat einen pH-Wert in einem Bereich von 0 bis 6. Es hat sich heraus gestellt, dass die Verbindungen dieser Erfindung bei den genannten pH-Werten lagerfähig sind.

### Bevorzugte Verbindungen

Nachfolgend werden einige besonders bevorzugte Strukturen dieser Erfindung beschrieben.

### Verbindung 3 - QuartCOSS

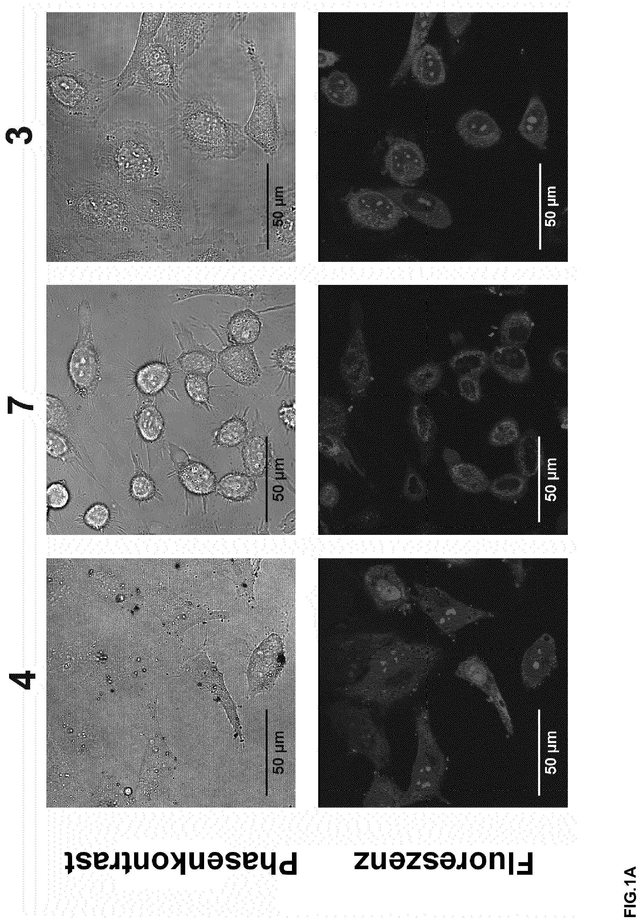

Eine bevorzugte Verbindung dieser Erfindung (Verbindung 3, QuartCOSS) hat folgende Strukturformel, wobei R eine Cargo-Molekülstruktur enthält, wie sie zuvor beschrieben wurde (insbesondere Strukturgruppe Y-Z). In diesem Molekül weisen sieben Reste aus R1 bis R8 eine quartäre Ammoniumgruppe auf. Die sieben Reste bestehen aus der Strukturgruppe X-A, wobei X unverzweigtes, nicht-zyklisches, unsubstituiertes, gesättigtes C₃-Alkylen ist. Die Verbindung 3 zeichnet sich durch eine besonders ausgeprägte Aufnahme in Zellen aus (FIG.1A, 2, 3, 4).

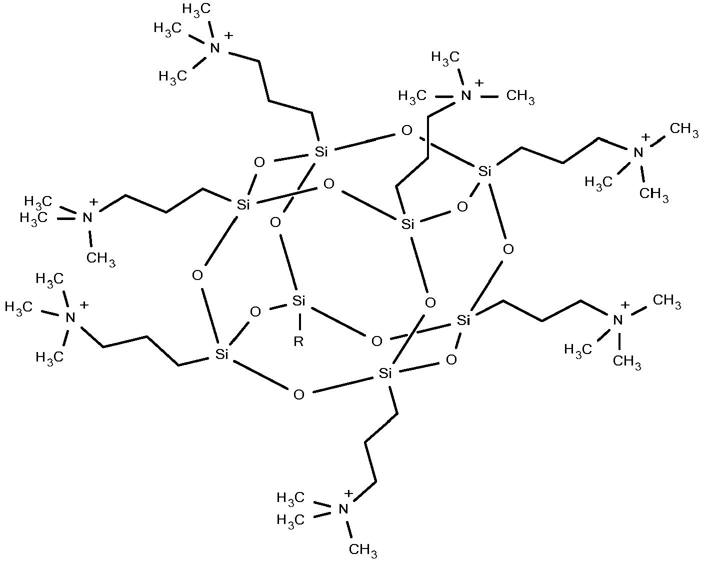

### Verbindung 4 - GuCOSS

Eine bevorzugte Verbindung dieser Erfindung (Verbindung 4, GuCOSS) hat folgende Strukturformel, wobei R eine Cargo-Molekülstruktur enthält, wie sie zuvor beschrieben wurde (insbesondere Strukturgruppe Y-Z). In diesem Molekül weisen sieben Reste aus R1 bis R8 eine Guanidingruppe auf. Die sieben Reste bestehen aus der Strukturgruppe X-A, wobei X unverzweigtes, nicht-zyklisches, unsubstituiertes, gesättigtes C₃-Alkylen ist. Die Verbindung 4 zeichnet sich durch eine besonders ausgeprägte Aufnahme in Zellen aus (FIG.1A, 2, 3, 4).

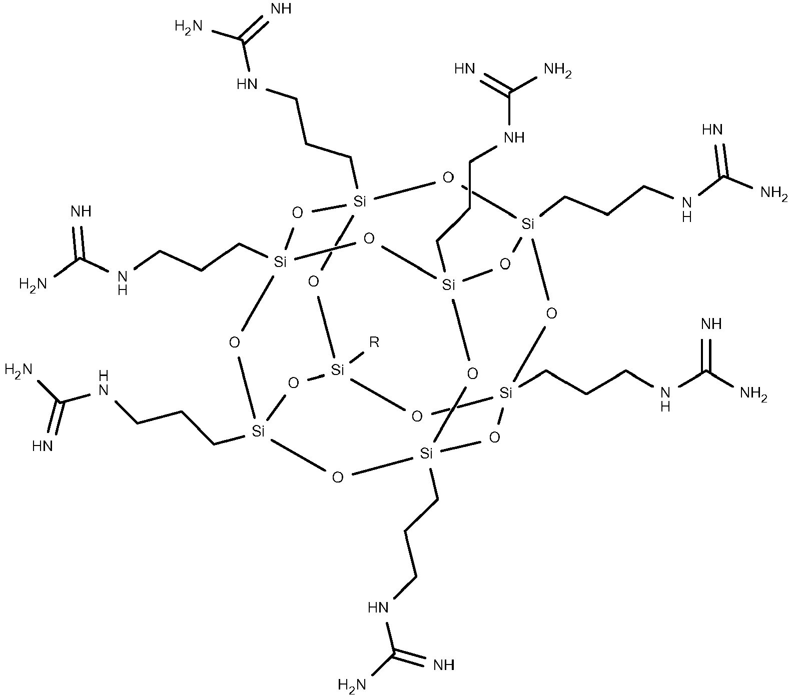

### Verbindung 6 - QuartCOSS-L

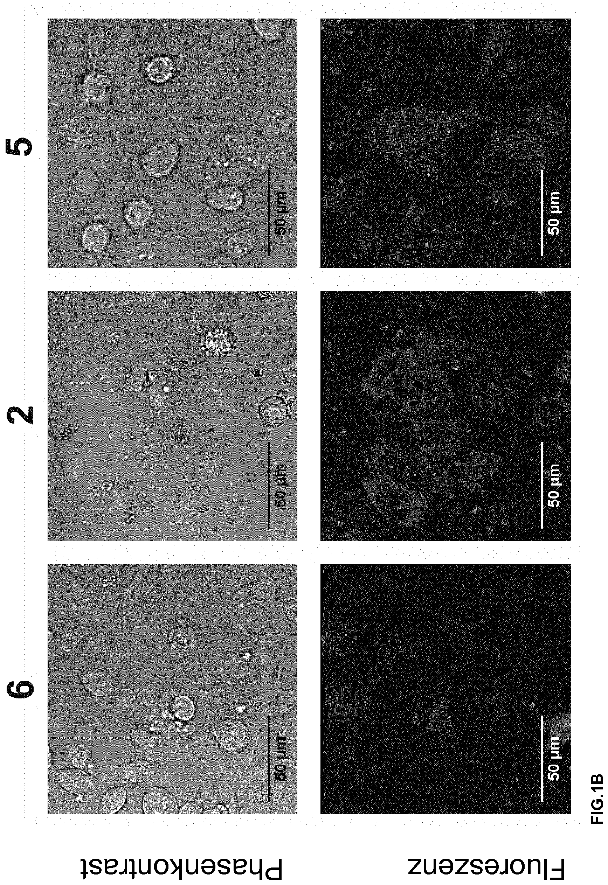

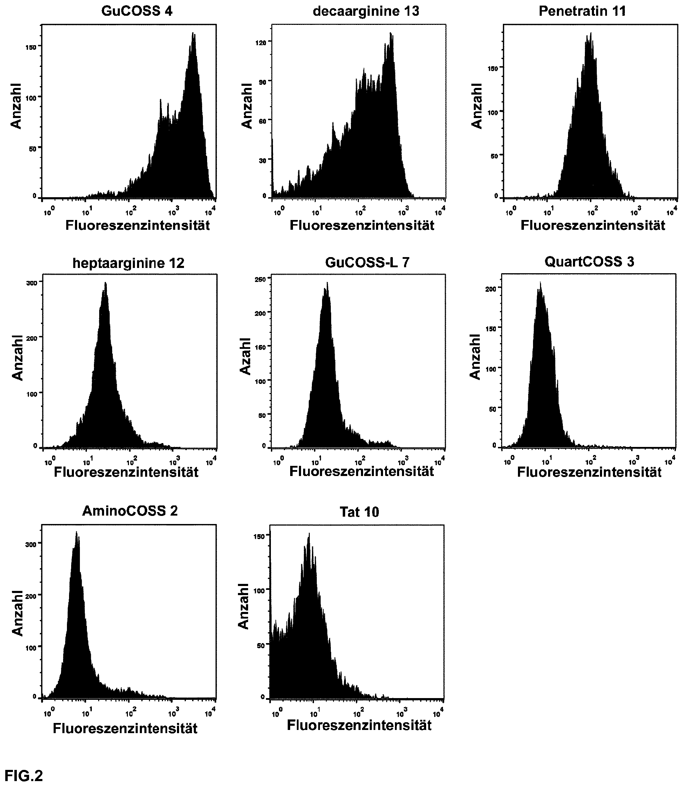

Eine bevorzugte Verbindung dieser Erfindung (Verbindung 6, QuartCOSS-L) hat folgende Strukturformel, wobei R eine Cargo-Molekülstruktur enthält, wie sie zuvor beschrieben wurde (insbesondere Strukturgruppe Y-Z). In diesem Molekül weisen sieben Reste aus R1 bis R8 eine quartäre Ammoniumgruppe auf. Die sieben Reste bestehen aus der Strukturgruppe X-A, worin X als X₁-L-X₂ gewählt ist. Darin sind X₁ und X₂ unverzweigtes, nicht-zyklisches, unsubstituiertes, gesättigtes C₃-Alkylen und L ist Aminocarbonyl. Die Verbindung 6 zeichnet sich durch eine besonders ausgeprägte Aufnahme in Zellen aus (FIG.1B, 2).

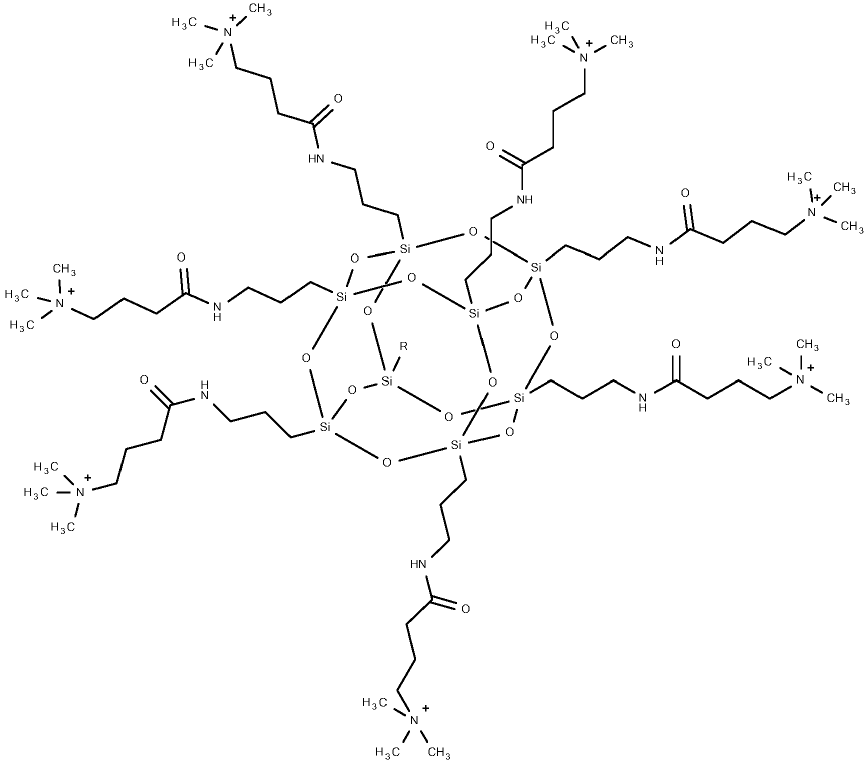

### Verbindung 7 - GuCOSS-L

Eine bevorzugte Verbindung dieser Erfindung (Verbindung 7, GuCOSS-L) hat folgende Strukturformel, wobei R eine Cargo-Molekülstruktur enthält, wie sie zuvor beschrieben wurde (insbesondere Strukturgruppe Y-Z). In diesem Molekül weisen sieben Reste aus R1 bis R8 eine Guanidingruppen auf. Die sieben Reste bestehen aus der Strukturgruppe X-A, worin X als X₁-L-X₂ gewählt ist. Darin sind X₁ und X₂ unverzweigtes, nicht-zyklisches, unsubstituiertes, gesättigtes C₃-Alkylen und L ist Aminocarbonyl. Die Verbindung 7 zeichnet sich durch eine besonders ausgeprägte Aufnahme in Zellen aus (FIG.1A, 2, 3).

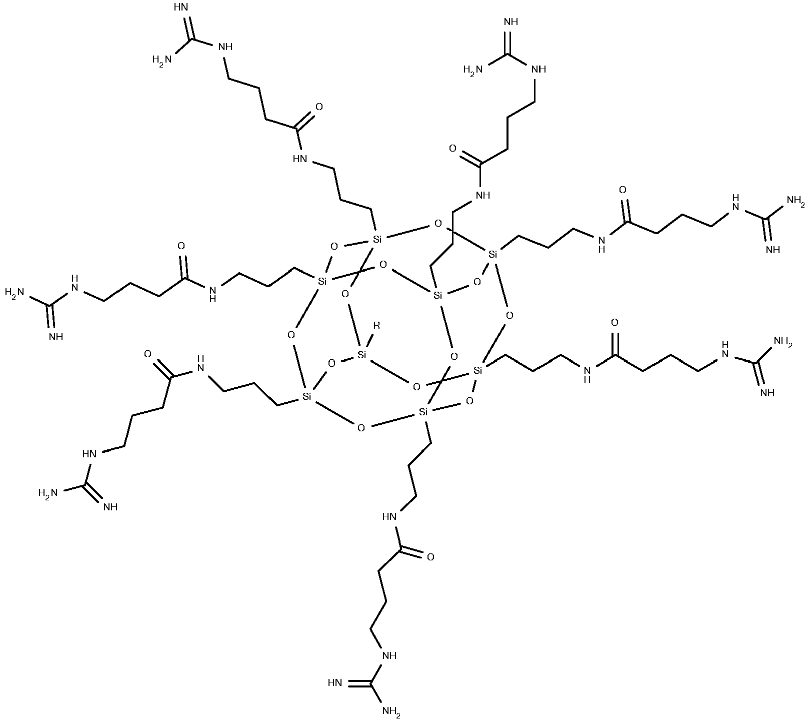

Die vorgestellten vier Verbindungen sind bevorzugte Ausgestaltungen der erfindungsgemäßen Verbindungen. Es wurde gezeigt, dass diese Verbindungen geeignet sind, Cargo-Moleküle über Zellmembranen hinweg zu transportieren. Die Strukturen sind an den Positionen R1 bis R8 sehr unterschiedlich substituiert. Insbesondere weisen die Substituenten stark unterschiedliche Kettenlängen auf.

### Herstellungsverfahren

Erfindungsgemäß ist auch ein Verfahren zur Herstellung einer Verbindung gemäß dieser Erfindung. In dem Verfahren sind Reste gleicher Benennung (mit Ausnahme von "R") identisch mit den zuvor für die Verbindungen beschriebenen Resten. Das Verfahren umfasst die folgenden Schritte:
a.) Bereitstellen eines Edukts E6, E8, E10 oder E12 mit nachfolgender Struktur: 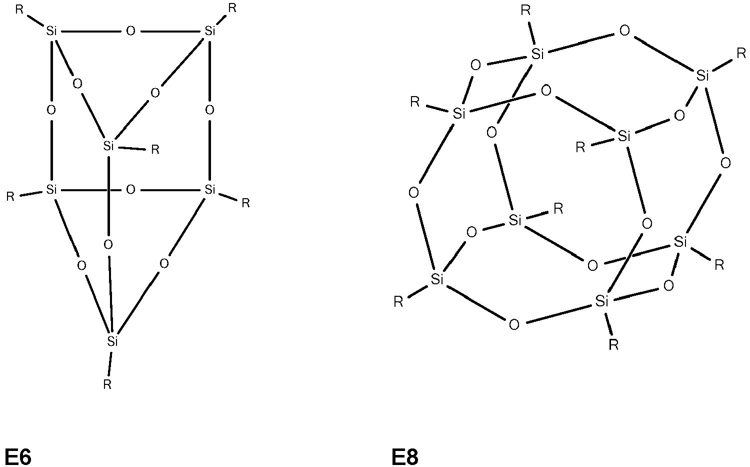 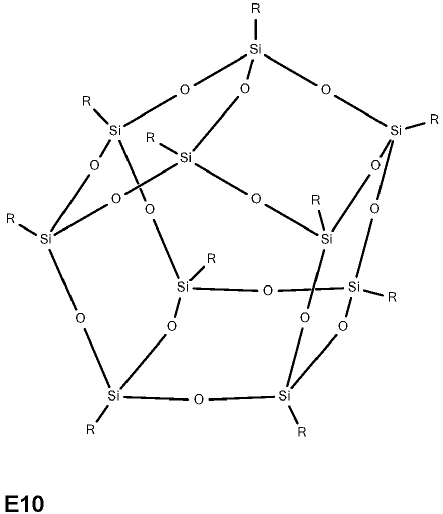 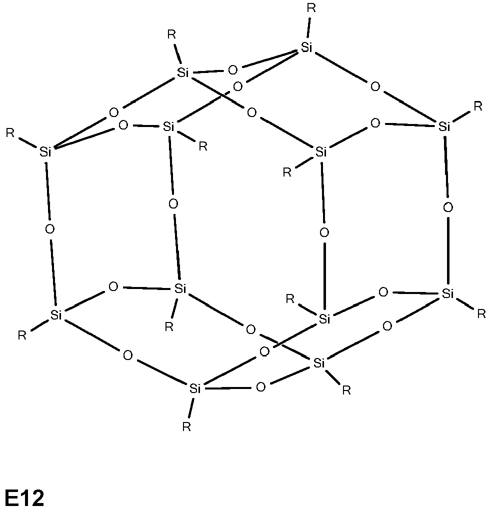 wobei wenigstens einer, insbesondere alle, der Reste R aus der Strukturgruppe X-A* besteht, wobei A* eine protonierte oder nicht protonierte Aminogruppe ist,
b.) optional Binden einer oder mehrerer Cargo-Molekülstrukturen an einen oder mehrere Reste R, insbesondere durch Reaktion der Cargo-Molekülstruktur mit A*,
c1.) Umsetzen des Edukts mit einem Alkylierungsmittel, um durch Alkylierung von A* eine quartäre Ammoniumverbindung zu erhalten, oder
c2.) Umsetzen des Edukts mit einem Guanidinierungsmittel, um durch Guanidinierung von A* eine Guanidingruppe zu erhalten.

Als Alkylierungsmittel wird vorzugsweise Methyliodid verwendet. Ein bevorzugtes Guanidinierungsmittel ist 1H-Pyrazol-1-carboximidamid-hydrochlorid.

Zur Herstellung der Verbindungen, die Reste mit der Strukturgruppe X₁-L-X₂ tragen, wird vorzugsweise eines der Edukte E6, E8, E10 bzw. E12 bereit gestellt, wobei wenigstens einer, insbesondere alle, der Reste R aus der Strukturgruppe X-A* besteht, wobei X der Gruppe X₁ entspricht. Das Edukt wird anstatt Schritt c1.) oder c2.) mit einem Reagens umgesetzt, welches das Strukturelement X₂-A oder X₂-A** trägt und unter Bildung der Gruppe L mit A* reagiert. A* ist insbesondere eine protonierte oder nicht protonierte Aminogruppe. Das Reagens ist vorzugsweise eine Substanz der Struktur HOOC-X₂-A. Geeignet sind neben der Carbonsäure HOOC-X₂-A auch Carbonsäureester und -halogenide. Ein bevorzugtes Reagens ist 4-Trimethylammoniumbuttersäure-NHS-ester.

Ein bevorzugtes Reagens mit der Strukturgruppe X₂-A** kann bei der Umsetzung eine Schutzgruppe tragen. Das Reagens ist vorzugsweise eine Substanz der Struktur HOOC-X₂-A**. Geeignet sind neben der Carbonsäure HOOC-X₂-A** auch Carbonsäureester und -halogenide. Ein bevorzugtes Reagens ist 4-Boc-Aminobuttersäure. Anschließend kann A** mit Hilfe eines Guanidinierungsmittels zu einer Guanidingruppe umgesetzt werden.

### Abbildungen

- **FIG.1**: zeigt mikroskopische Aufnahmen von HeLa-Zellen, die mit den erfindungsgemäßen Verbindungen inkubiert wurden.
- **FIG.2**: zeigt Ergebnisse einer Durchflusszytometrieuntersuchung an inkubierten HeLa-Zellen.
- **FIG.3**: zeigt Ergebnisse einer Durchflusszytometrieuntersuchung an inkubierten HEK-293-Zellen.
- **FIG.4**: zeigt Ergebnisse einer Durchflusszytometrieuntersuchung an inkubierten CHO-Zellen.
- **FIG.5**: zeigt einen Vergleich der Fluoreszenzintensitäten bei Verwendung unterschiedlicher Verbindungen.
- **FIG.6**: Darstellung der Zelltoxizität von Verbindungen dieser Erfindung im Vergleich zu freiem Doxorubicin.

### Beispiele

### Lösungsmittel

Die Lösungsmittel wurden von Karl Roth GmbH (Karlsruhe, Deutschland), Acros (Taufkirchen, Deutschland), Fluka (Buchs, Schweiz) oder Sigma-Aldrich (St. Louis, USA) bezogen. Es wurden die folgenden Qualitäten gewählt: Aceton: Synthesequalität; Dimethylsulfoxid (DMSO): Spektroskopiequalität; Acetonitril (MeCN): HPLC-Qualität. Wasser in Millipore-Qualität wurde für die HPLC-Analyse verwendet. Einige Lösungsmittel wurden getrocknet und gereinigt nach folgenden Verfahren:
MeCN: 1 g/L Natriumhydroxid-Dispersion wurde dem Lösungsmittel hinzu gegeben. Nach Erhitzen unter Rückfluss für eine Stunde wurde das Lösungsmittel destilliert. 2 g/L P₂O₅ wurden hinzu gegeben und die Mischung wurde unter Rückfluss für eine Stunde erhitzt. Die Mischung wurde dann destilliert und im Dunkeln unter Argonatmosphäre über einem aktivierten 4 Å Molekularsieb gelagert.
DMSO: Das Lösungsmittel wurde für einen Tag bei Raumtemperatur über 25 g/L Calciumhydrid vorgetrocknet. Danach wurde es destilliert und im Dunkeln unter Argonatmosphäre über einem aktivierten 4 Å Molekularsieb gelagert.
*N*,*N*-Diisopropylethylamin (DIEA): Das Lösungsmittel wurde über 50 g/L Kaliumhydroxid für eine Stunde unter Rückfluss erhitzt. Nach Abkühlen auf Raumtemperatur wurde es dekantiert und 50 g/L frisches Kaliumhydroxid wurden hinzu gegeben. Die Mischung wurde über für eine Stunde unter Rückfluss erhitzt und danach dekantiert. Es wurde erneut destilliert und das erhaltene Lösungsmittel wurde für drei Stunden über Calciumhydrid unter Rückfluss erhitzt. Danach wurde es destilliert und im Dunkeln unter Argonatmosphäre über einem aktivierten 4 Å Molekularsieb gelagert.

### Reagenzien

Alle Reagenzien wurden wie von Sigma-Aldrich oder Acros Organics geliefert verwendet, ohne weitere Reinigung oder Trocknung. Aminosäuren und Harze wurden von Novabiochem (Marke von Merck KGaA, Darmstadt, Deutschland), oder Iris Biotech (Marktredwitz, Deutschland) bezogen.

### Massenspekrometrie

ESI-MS-Spektren wurden unter Verwendung eines Shimadzu LCMS-2020 Massenspektrometers erhalten, welches mit einer Phenomenex Jupiter 5u C4 LC-Säule (50x1 mm, 5 µm, 300 Å) ausgestattet war. Das Elutionssystem bestand aus 0,1 Vol-% wässriger Ameisensäure (LC-MS-Qualität, Sigma-Aldrich, St. Louis, USA) als Eluent A und 100% Acetonitril enthaltend 0,1 Vol-% Ameisensäure (LC-MS-Qualität, Karl Roth GmbH, Karlsruhe, Deutschland) als Eluent B.

### Flüssigchromatographie

Die analytische RP-HPLC wurde mit einem Varian 920 LC durchgeführt, welche mit einer Phenomenex Luna Hypersil 5u BDS C18 LC-Säule (5 u, 130 Å, 150x4, 60 mm, 5 µm) ausgestattet war. Die Flussrate betrug 1 mL/min. Für die Isolation von Peptiden wurde eine semi-präparative RP-HPLC mit einem Varian 940 LC durchgeführt, welche mit einer präparativen C18-Säule (Phenomenex Luna 5u C18, 250x20 mm, S-4 µm, 8 nm) ausgestattet war. Die Flussrate betrug 18 mL/min. Als Eluent A wurde 0,1 Vol-% wässrige TFA gewählt, als Eluent B 90 Vol-% wässriges MeCN mit 0,1 Vol-% TFA. Einer 5-minütigen isokratischen Elution (Anfangskonzentration von Eluent B) folgte eine 20-minütige Gradientenelution. Die Absorption wurde mit einem UV/vis-Detektor bei 220 nm und 280 nm oder 220 nm und 534 nm gemessen.

### NMR

¹H, ¹³C, ²⁹Si und die 2D NMR-Spektren ¹H-¹³C HSQC, ¹H-¹H COSY und ¹H-²⁹Si HMBC wurden mit einem *Bruker DRX 500 (Bruker Corporation,* Billerica, USA) (500 MHz) aufgenommen. Alle Proben wurden in deuteriertem DMSO-d₆ gelöst, welches von *Euriso Top* (Gif-Sur-Yvette, France) bezogen wurde.

In einem ersten Schritt der Strukturaufklärung wurden ¹H und ²⁹Si-Spektren analysiert. Die chemische Verschiebung der Si-Kerne in dem käfigähnlichen Kern des Nanopartikels wurde als Indikator benutzt. Wenn eine geschlossene Struktur vorhanden ist, in der alle Si-Kerne mit drei anderen Si-Kernen über Sauerstoffatome verbunden sind (bezeichnet als T₈-Struktur), dann wird die chemische Verschiebung der vier nicht-isochronen Si-Einheiten bei etwa -65 ppm erwartet. Hydrolyseprodukte (oder andere Zersetzungsprodukte) werden bei etwa -40 ppm erwartet. Die Spektren bestätigen die Integrität der käfigähnlichen Struktur und die asymmetrische Substitution ermöglicht in bevorzugten Konstellationen eine vollständige Zuordnung aller nicht-isochronen Resonanzen, wobei Resonanzüberschneidungen minimal sind.

### Synthese von TAMRA-heptaammonium-COSS 2 (AminoCOSS)

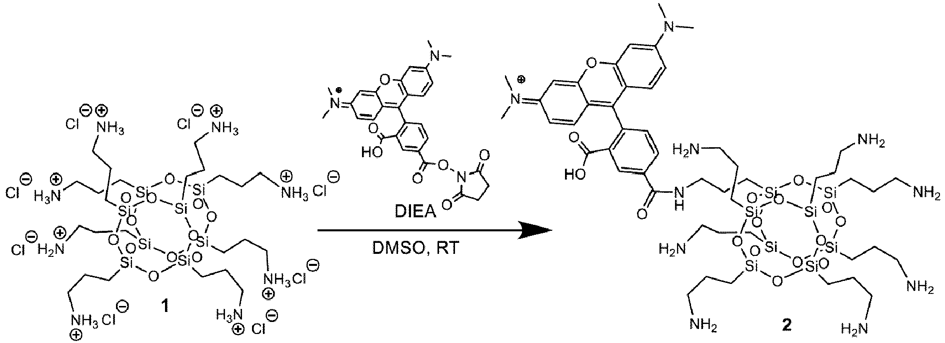

Eine Mischung aus 7 mg (13,2 µmol, 1 Äquivalent) TAMRA-NHS-Ester und 13,8 µL (79,5 µmol, 6 Äquivalente) trockenes DIEA wurden in 8 mL trockenem DMSO gelöst. 100 mg (85,2 µmol, 6,4 Äquivalente) Octaammonium-POSS-Hydrochlorid 1 (bezogen von Hybrid Plastics Inc., USA) wurden in 0,5 mL trockenem DMSO gelöst und heftig gerührt. Die zuvor beschriebene Mischung wurde langsam unter Verwendung einer peristaltischen Pumpe hinzu gegeben (Flussrate: 0,05 mL/min). Die Mischung wurde für weitere 2 Stunden bei Raumtemperatur gerührt. Danach wurde das Lösungsmittel durch Lyophilisation entfernt. Der violette Rückstand wurde in trockenem Acetonitril suspendiert und der unlösliche Teil wurde dreimal mit trockenem Acetonitril gewaschen. Der Niederschlag wurde in 10 Vol-% wässrigem MeCN mit 0,1 Vol-% TFA gelöst und mittels semi-präparativer RP-HPLC aufgereinigt (Gradient 10 bis 40). Nach Lyophilisation wurden 13 mg eines violetten Feststoffes erhalten (76,3%).
RP-HPLC, 10→40% B, t_{R} = 19,35 min
HR-MS Berechnung für C₄₉H₈₄N₁₀O₁₆Si₈ m/z: 431,8146; gemessen: 431,8134 [M+3H]³⁺
¹H NMR (500 MHz, DMSO-d₆) δ: 0,71 (h, J = 10,4, 8,5 Hz, 16H), 1,65 (h, J = 11,5, 9,3 Hz, 16H), 2,80 (dt, J = 13,7, 7,0 Hz, 16H), 3,23 (s, 12H), 6,95 (t, J = 20,6 Hz, 5H), 7,54 (d, J = 8,1 Hz, 1H), 7,98 - 8,03 (m, 21H), 8,30 (d, J = 8,0 Hz, 1H), 8,65 (s, 1H), 9,00 (t, J = 5,5 Hz, 1H).
²⁹Si NMR (99 MHz, DMSO-d₆) δ: -66,61, -66,63, -66,66.

### Synthese von TAMRA-heptatrimethylammonium-COSS 3 (QuartCOSS)

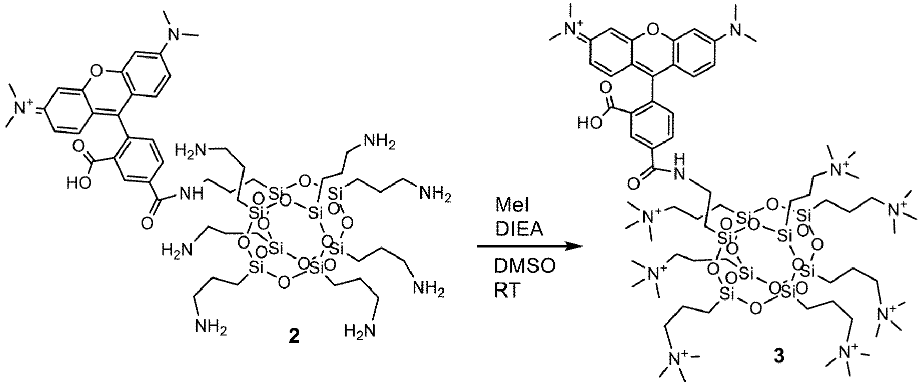

Eine Mischung aus 25 mg (19,3 µmol, 1 Äquivalent) TAMRA-gelabeltem AminoCOSS 2, 84 µL (482,8 µmol, 25 Äquivalente) trockenem DIEA und 42 µL (674,7 µmol, 35 Äquivalente) Methyliodid wurden in 5 mL trockenem DMSO gelöst. Nach 5-tägigem Rühren wurde das Lösungsmittel im Rotationsverdampfer entfernt. Der violette Rest wurde in 10 Vol-% wässrigem MeCN mit 0,1 Vol-% TFA gelöst und mittels semi-präparativer RP-HPLC gereinigt (Gradient 10 bis 40). Nach Lyophilisation wurden 14,9 mg eines violetten Feststoffes 3 erhalten (48,8%).
RP-HPLC, 10→40% B, t_{R} = 19,81 min
¹H NMR (500 MHz, DMSO-d₆) δ 0,54 - 0,84 (m, 16H), 1,59 - 1,93 (m, 16H), 3,08 (d, J = 7,2 Hz, 63H), 3,30 (d, J = 17,4 Hz, 28H), 6,86 - 7,15 (m, 5H), 7,63 (d, J = 8,0 Hz, 1H), 8,37 (d, J = 8,0 Hz, 1H), 8,71 (s, 1H), 9,11 - 9,45 (m, 1H).
²⁹Si NMR (99 MHz, DMSO-d₆) δ: -67,26, -67,30, -67,39.

### Synthese von TAMRA-heptaguanidin-COSS 4 (GuCOSS)

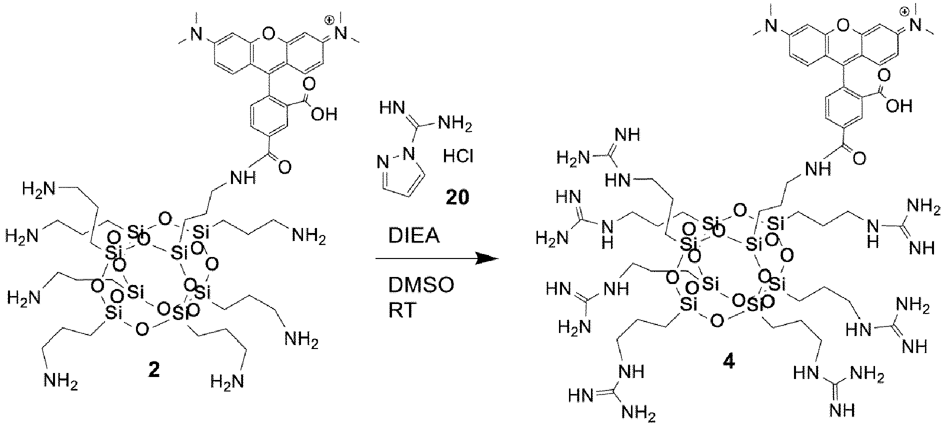

19,2 mg von TAMRA-gelabeltem AminoCOSS 2 (1 Äquivalent, 14,8 µmol), 45,6 mg 1H-Pyrazol-1-carboximidamid-hydrochlorid (21 Äquivalente, 311,4 µmol) und 126 µL DIEA (50 Äquivalente, 741 µmol) wurden in 5 mL trockenem DMSO gelöst und bei Raumtemperatur für 4 Tage gerührt. Die Mischung wurde gefriergetrocknet. Der ölige Rückstand wurde in trockenem Acetonitril suspendiert und der unlösliche Anteil wurde zweimal mit trockenem Acetonitril gewaschen. Der violette Feststoff wurde in 10 Vol-% wässrigem MeCN mit 0,1 Vol-% TFA gelöst und mittels semi-präparativer RP-HPLC gereinigt (Gradient 10 bis 100). Nach Lyophilisation wurden 5,5 mg eines violetten Feststoffes **4** erhalten.
RP-HPLC, 10→40% B, t_{R} = 21,50 min
HR-MS Berechnung für C₅₆H₉₈N₂₄O₁₆Si₈ m/z: 318,3222; gemessen: 318,3221 [M+5H]⁵⁺
¹H NMR (500 MHz, DMSO-d₆) δ: 0,64 (dd, J = 11,0, 6,0 Hz, 16H), 1,53 (q, J = 7,9 Hz, 16H), 2,54 (s, 8H), 3,09 (q, J = 6,5 Hz, 30H), 6,87 - 7,58 (bs, η-NH2 Gua), 6,96 (d, J = 30,9 Hz, 5H), 7,84 (q, J = 6,4 Hz, 9H, ε-NH Gua), 8,25 (s, 1H), 8,58 (s, 1H), 8,91 (s, 1H).
²⁹Si NMR (99 MHz, DMSO-d₆) δ: -66,42.

### Synthese von TAMRA-hepta-4-aminobuttersäure-COSS-amid 5 (AminoCOSS-L)

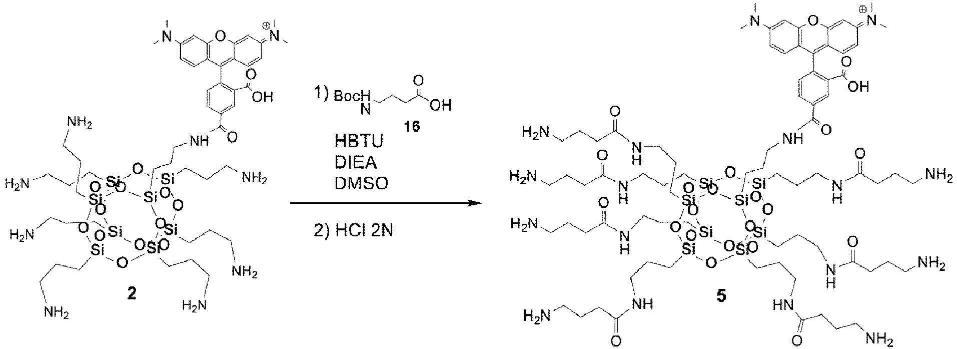

Eine Mischung aus 65,9 mg (324,3 µmol, 21 Äquivalente) 4-Boc-Aminobuttersäure (Sigma-Aldrich) 16, 134,6 µL (772,2 µmol, 50 Äquivalente) trockenem DIEA und 117,2 mg (308,9 µmol, 20 Äquivalente) HBTU wurden in 4 mL trockenem DMSO gelöst und für 10 Minuten bei Raumtemperatur voraktiviert. 20 mg (15,4 µmol, 1 Äquivalent) TAMRA-gelabeltes AminoCOSS 2 wurde hinzugefügt und die Mischung für 2 Stunden gerührt. Das Lösungsmittel wurde durch Lyophilisation entfernt. Entschützung des violetten Rückstandes wurde mit 50 Vol-% wässriger TFA über 7 Stunden durchgeführt. Das Lösungsmittel wurde mit einem Rotationsverdampfer entfernt und der Rückstand wurde in 10 Vol-% wässrigem MeCN mit 0,1 Vol-% TFA gelöst und mittels semi-präparativer RP-HPLC gereinigt (Gradient 10 bis 40). Nach Lyophilisation wurden 18,3 mg eines violetten Feststoffes **5** erhalten (45,7%).
RP-HPLC, 10→40% B, t_{R} = 19,82 min
HR-MS Berechnung für C₇₇H₁₃₃N₁₇O₂₃Si₈ m/z: 378,5656; gemessen: 378,5661 [M+5H]⁵⁺
¹H NMR (500 MHz, DMSO-d₆) δ: 0,42 - 0,72 (m, 16H), 1,25 (q, J = 7,4 Hz, 14H), 1,46 (q, J = 7,9 Hz, 14H), 1,64 - 1,85 (m, 16H), 2,12 - 2,24 (m, 14H), 2,78 (m, 16H), 3,02 (t, J = 6,8 Hz, 12H), 6,65 (m, J = 60,3 Hz, 5H), 7,40 (s, 1H), 7,94 (d, J = 34,3 Hz, 29H), 8,17 - 8,27 (s, 1H), 8,54 (d, J = 19,5 Hz, 1H), 8,92 (d, J = 8,6 Hz, 1H).
²⁹Si NMR (99 MHz, DMSO-d₆) δ: -66,23, -66,25.

### Synthese von TAMRA-hepta-4-trimethylammoniumbuttersäure-COSS-amid 6 (QuartCOSS-L)

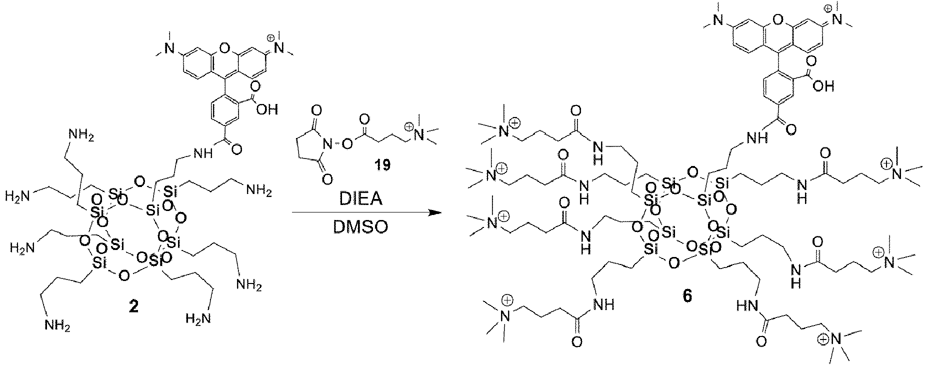

Eine Mischung aus 25 mg (19,3 µmol, 1 Äquivalent) TAMRA-gelabeltem AminoCOSS 2, 98,6 mg (324,3 µmol, 21 Äquivalente) 4-Trimethylammoniumbuttersäure-NHS-ester **19** und 134,4 µL (772,2 µmol, 50 Äquivalente) trockenes DIEA wurden in 5 mL trockenem DMSO gelöst und für 8 Tage gerührt. Das Lösungsmittel wurde mittels Lyophilisation entfernt. Der Rückstand wurde in 10 Vol-% wässrigem MeCN mit 0,1 Vol-% TFA gelöst und mittels semi-präparativer RP-HPLC gereinigt (Gradient 10 bis 40). Nach Lyophilisation wurden 18,3 mg eines violetten Feststoffes **6** erhalten (45,7%).
RP-HPLC, 10→40% B, t_{R} = 18,87 min
HR-MS Berechnung für C₉₈H₁₈₂N₁₇O₂₃Si₈ m/z: 312,7387; gemessen: 312,7384 [M]⁷⁺
¹H NMR (500 MHz, DMSO-d₆) δ: 0,62 (q, J = 8,3 Hz, 16H), 1,47 (h, J = 7,5 Hz, 16H), 1,82 - 1,96 (m, 16H), 2,15 (t, J = 7,3 Hz, 16H), 3,03 - 3,07 (m, 77H), 3,17 - 3,31 (m, 30H), 6,96 (d, J = 30,9 Hz, 5H), 7,55 (d, J = 8,0 Hz, 1H), 8,04 (td, J = 5,8, 3,3 Hz, 8H), 8,31 (dd, J = 8,0, 1,8 Hz, 1H), 8,67 (s, 1H), 8,96 - 9,05 (m, 1H).
²⁹Si NMR (99 MHz, DMSO-d₆) δ: -66,24.

### Synthese von TAMRA-hepta-4-guanidinobuttersäure-COSS-amid 7 (GuCOSS-L)

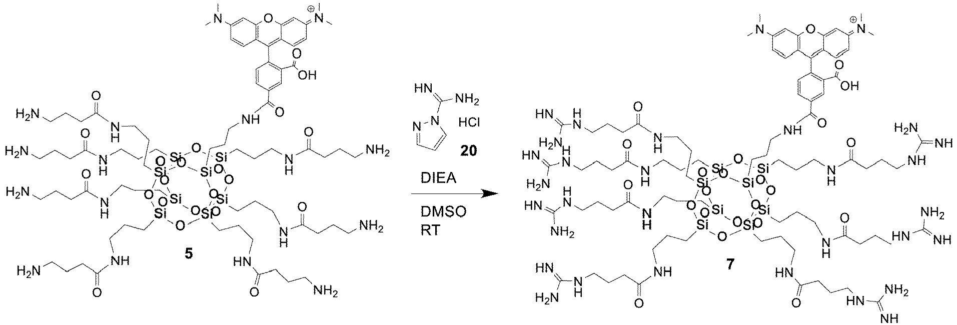

Eine Mischung aus 12 mg (6,4 µmol, 1 Äquivalent) TAMRA-gelabeltem hepta-4-aminobuttersäure-COSS-amid **5,** 44.1 µL (253,9 µmol, 40 Äquivalente) trockenem DIEA und 22,3 mg (152,4 µmol, 24 Äquivalente) 1H-Pyrazol-carboximidamid-hydrochlorid **20** wurde in 10 mL trockenem DMSO gelöst und für 8 Tage gerührt. Das Lösungsmittel wurde mittels Lyophilisation entfernt. Der Rückstand wurde in 10 Vol-% wässrigem MeCN mit 0,1 Vol-% TFA gelöst und mittels semi-präparativer RP-HPLC gereinigt (Gradient 10 bis 40). Nach Lyophilisation wurden 3,6 mg eines violetten Feststoffes **7** erhalten (26%).
RP-HPLC, 10→40% B, t_{R} = 20,9 min
HR-MS Berechnung für C₈₄H₁₄₇N₃₁O₂₃Si₈ m/z: 364,6646; gemessen: 364,6643 [M+6H]⁶⁺
¹H NMR (500 MHz, DMSO-d₆) δ: 0,60 (d, J = 8,4 Hz, 16H), 1,25 (q, J = 7,2 Hz, 16H), 1,46 (q, J = 7,8 Hz, 14H), 1,66 (t, J = 6,7 Hz, 16H), 2,13 (d, J = 7,4 Hz, 14H), 3,04 (dt, J = 25,2, 6,9 Hz, 14H), 3,26 (s, 12H), 6,90 - 7,64 (m, 6H), 7,73 - 7,86 (m, 14H), 7,94 (t, J = 5,2 Hz, 7H), 8,36 (s, 1H), 8,68 (s, 1H), 8,94 (s, 1H).
²⁹Si NMR (99 MHz, DMSO-d₆) δ: -66,23, -66,25.

### Synthese von Fluorescein-TAMRA-hexaguanidin-COSS 8

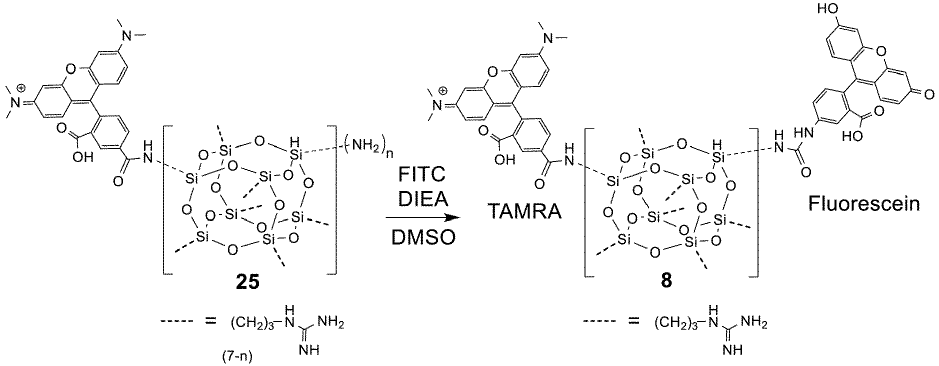

20 mg TAMRA-gelabeltes AminoCOSS **2** (1 Äquivalent, 15,4 µmol), 47,5 mg 1H-Pyrazol-1-carboximidamid-hydrochlorid (21 Äquivalente, 324,2 µmol) und 99,5 mg (134,5 µL, 50 Äquivalente, 770 µmol) trockenes DIEA wurden in 5 mL trockenem DMSO gelöst und für drei Tage bei Raumtemperatur gerührt. Die Bildung von sechs- und siebenfach guanyliertem COSS wurde mit LC-MS der Reaktionsmischung bestätigt. Die Mischung wurde gefriergetrocknet und der ölige Rückstand wurde in trockenem Acetonitril suspendiert. Der unlösliche Anteil wurde dreimal mit trockenem Acetonitril gewaschen. Der violette Feststoff wurde in 10 Vol-% wässrigem MeCN mit 0,1 Vol-% TFA gelöst und mittels semi-präparativer RP-HPLC gereinigt (Gradient 10 bis 100). Nach Lyophilisation wurden 5,6 mg einer Mischung aus Verbindung **25** und TAMRA-Heptaguanidin-COSS **4** (GuCOSS) erhalten. Die Produktmischung wurde mit 2,7 mg Fluoresceinisothiocyanat (FITC, Applichem, 2 Äquivalente, 6,9 µmol) und 1,8 mg trockenem DIEA (2,4 µL, 4 Äquivalente, 13,9 µmol) in 2 mL trockenem DMSO bei Raumtemperatur über Nacht umgesetzt. Das Lösungsmittel wurde mittels Lyophilisation entfernt und der ölige Rückstand wurde mit HPLC gereinigt. Nach Lyophilisation wurden 1,9 mg eines roten Feststoffes erhalten (6,6%).
RP-HPLC, 10→100% B, t_{R} = 14,57 min
HR-MS Berechnung für C₇₆H₁₀₇N₂₃O₂₁SSi₈ m/z: 645,5368; gemessen: 645,5362 [M+3H]³⁺

### Synthese von Mono-4-Aminopropylamid-heptaguanidin-COSS 9

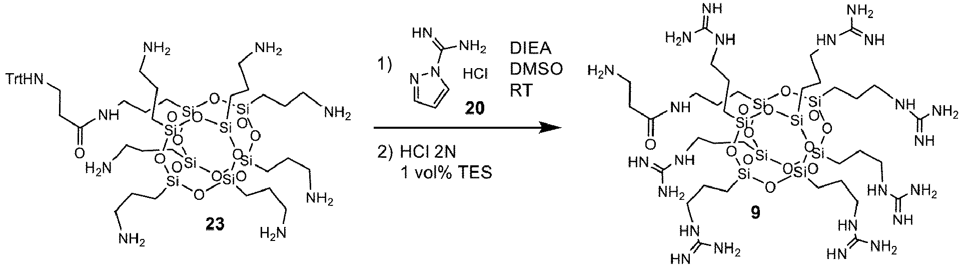

123 mg (0,103 mmol, 1 Äquivalent) Trityl-geschütztes 4-Aminopropylamid-heptaammonium-COSS **23,** 317 mg (2,16 mmol, 21 Äquivalente) 1H-Pyridazol-1-carboxamid-Hydrochlorid **20** und 666 mg (5,2 Äquivalente, 897 µL) trockenes DIEA wurden in 5 mL trockenem DMSO gemischt und die Mischung wurde für zwei Tage bei Raumtemperatur gerührt. Danach wurde das Lösungsmittel mittels Lyophilisation entfernt und der ölige Rückstand wurde in trockenem Acetonitril suspendiert. Der unlösliche Anteil wurde zweimal mit trockenem Acetonitril gewaschen. Der Niederschlag wurde in 5 Vol-% wässriger AcOH gewaschen und mittels semi-präparativer RP-HPLC mit 0,1 Vol-% AcOH gereinigt (Gradient 0 bis 60). Nach Lyophilisation wurden 44,5 mg eines farblosen Trityl-geschützten Zwischenproduktes 24 erhalten (29%). Zum Entschützen der Trityl-Schutzgruppe wurden 1 mL 2N HCl und 1 mL CHCl₃ hinzugefügt und die Lösung für 3 Stunden bei Umgebungstemperatur geschüttelt. Die organische Phase wurde entfernt und die wässrige Phase wurde zweimal mit je 1 mL CHCl₃ extrahiert. Die kombinierten organischen Phasen wurden verworfen und 9 mL deionisiertes Wasser wurde der wässrigen Phase hinzugefügt. Das Lösungsmittel wurde mittels Lyophilisation entfernt. Der verbleibende, farblose Feststoff wurde in 5 mL einer 5 Vol-% wässrigen AcOH gelöst und das Lösungsmittel wurde mittels Lyophilisation entfernt. 37,1 mg eines farblosen Feststoffes 9 wurden erhalten (99%).

### Protected 24:

ESI-MS Berechnung für C₅₃H₉₇N₂₃O₁₃Si₈ m/z: 745,58 gemessen 745,61 [M+2H]²⁺, berechnet 497,39 gemessen 497,32 [M+3H]³⁺.
RP-HPLC, 10→60% B, t_{R}= 20,38 min.
¹H NMR (400 MHz, DMSO-d₆) δ: 0,36-0,80 (m, 16H), 1,50 (d, J = 11,8 Hz, 16H), 2,18 (d, J = 7,3 Hz, 2H), 2,24 - 2,37 (m, 2H), 2,92 - 3,19 (m, 16H), 7,18 (t, J = 7,2 Hz, 3H), 7,28 (d, J = 7,7 Hz, 6H), 7,37 (s, 6H), 7,62 (s, 35H), 8,50 (s, 8H).
²⁹Si NMR (79 MHz, DMSO) δ: -66,03, -66,21, -66,25, -66,32

### Deprotected 9:

¹H NMR (400 MHz, DMSO-d₆) δ: 0,63 (s, 16H), 1,49 (d, J = 24,2 Hz, 16H), 2,55 (s, 2H), 3,03 (d, J = 29,7 Hz, 2H), 3,22 (s, 16H), 6,82 - 7,69 (m, 35H), 7,96 (s, 8H).

### Synthese von β-Alanin(Trt) 22

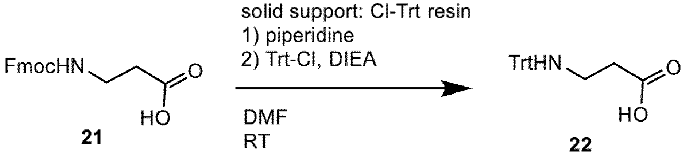

1 g 2-Chlorotritylharz (Iris Biotech GmbH) wurde für eine Stunde bei Raumtemperatur in trockenem DCM quellen gelassen. 0,995 g (3,2 mmol, 2 Äquivalente) Fmoc-β-Ala und 0,825 mg (6,4 mmol, 4 Äquivalente, 1,112 mL) trockenes DIEA wurden in 10 mL trockenem DCM hinzu gegeben und die Mischung wurde für 2 Stunden bei Raumtemperatur geschüttelt. Das Harz wurde fünfmal mit DCM gewaschen und danach dreimal unter Verwendung von jeweils 5 mL einer Mischung aus DCM, DIEA und MeOH (17:2:1, v:v:v). Die Fmoc-Schutzgruppe wurde durch doppelte Entschützung entfernt (20 Vol-% Piperidin in DMF). Das Harz wurde mit DCM und 1,784 g (6,4 mmol, 4 Äquivalente) gewaschen und 1,238 g (9,6 mmol, 6 Äquivalente, 1,674 mL) trockenes DIEA wurden in 10 mL trockenem DCM hinzugefügt. Die Mischung wurde über Nacht bei Raumtemperatur geschüttelt. Das Harz wurde fünfmal mit DCM gewaschen. Trityl-geschütztes β-Alanin 22 wurde von dem Harz gespalten unter Verwendung von 10 mL einer Mischung aus Essigsäure, Methanol und DCM (5:1:4, v:v:v). Die Mischung wurde drei Stunden bei Raumtemperatur geschüttelt. Das Lösungsmittel wurde mit einem Rotationsverdampfer entfernt. 0,496 g eines weißen Feststoffes 22 wurden erhalten (93,5%) und ohne weitere Aufreinigung verwendet.
ESI-MS Berechnung für C₂₂H₂₁NO₂ m/z: 331,42, gemessen 330,06 [M-H]⁻.
¹H NMR (300 MHz, DMSO-d₆) δ: 2,20 (t, J = 6,7 Hz, 2H), 2,44 (t, J = 6,4 Hz, 2H), 2,95 (s, 1H), 7,19 (t, J = 7,3 Hz, 3H), 7,30 (t, J = 7,9 Hz, 6H), 7,41 (d, J = 7,2 Hz, 6H).
¹³C-NMR (75 MHz, DMSO-d₆) δ: 34,75, 39,40, 70,29, 126,05, 127,67, 128,31, 145,96, 173,77.

### Synthese von Trityl-geschütztem Mono-4-aminopropylamidheptaamino-COSS 23

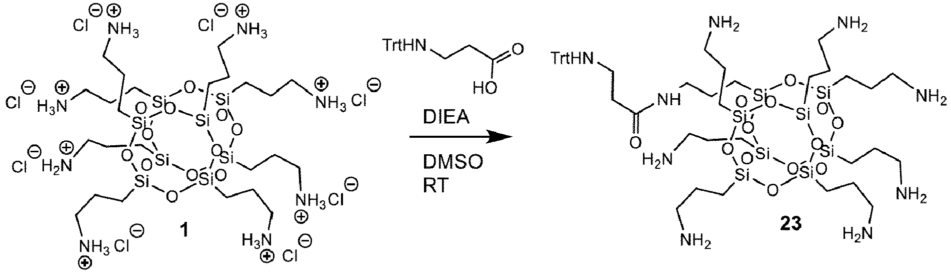

0,104 g (1,1 Äquivalente, 0,312 mmol) β-Alanin(Trt) **22,** 0,148 g (1 Äquivalent, 0,284 mmol) Benzotriazol-1-yl-oxytripyrrolidinophosphonium-hexafluorophosphat (PyBOP) und 73,4 mg (2 Äquivalente, 0,568 mmol, 98,9 mL) trockenes DIEA wurden in 5 mL trockenem DMSO gelöst und für 10 Minuten bei Raumtemperatur geschüttelt. Nach 10 Minuten wurde das Volumen mit trockenem DMSO auf 40 mL erhöht. 1 g Octaammonium-POSS-Hydrochlorid **1** wurde in 5 mL trockenem DMSO gelöst und die vorgenannte Lösung wurde langsam mit einer peristaltischen Pumpe zu der heftig gerührten Lösung gegeben (Flussrate: 0,05 mL/min). Die Mischung wurde weitere 2 Stunden bei Raumtemperatur gerührt. Danach wurde das Lösungsmittel mittels Lyophilisation entfernt. Der ölige Rückstand wurde in trockenem Acetonitril suspendiert und der unlösliche Anteil wurde zweimal mit trockenem Acetonitril gewaschen. Der Niederschlag wurde in 10 Vol-% wässrigem MeCN mit 0,1 Vol-% AcOH gelöst und mittels semi-präparativer RP-HPLC gereinigt (Gradient 10 bis 100). Nach der Lyophilisation wurden 123 mg eines farblosen Feststoffes 23 erhalten (33%).
RP-HPLC, 10→100% B, t_{R}= 14,82 min.
ESI-MS Berechnung für C₄₆H₈₃N₉O₁₃Si₈ m/z: 1194,90 gemessen 1194,63 [M+H]⁺, berechnet 598,45 gemessen 598,26 [M+2H]²⁺.
¹H NMR (400 MHz, DMSO-d₆) δ: 0,44-0,86 (m, 16H), 1,47 (q, J = 8,5, 8,0 Hz, 2H), 1,73 (dt, J = 16,7, 8,6 Hz, 16H), 2,61 - 2,92 (m, 16H), 2,99 (s, 2H), 7,34 (d, J = 51,7 Hz, 15H), 8,12 - 8,44 (m, 21H), 10,76 (s, 1H).
²⁹Si NMR (79 MHz, DMSO) δ: -65,99, -66,49, -66,52, -66,62.

### Synthese von Fmoc-Cys(Trt)-heptaammonium-COSS (Cys-COSS)

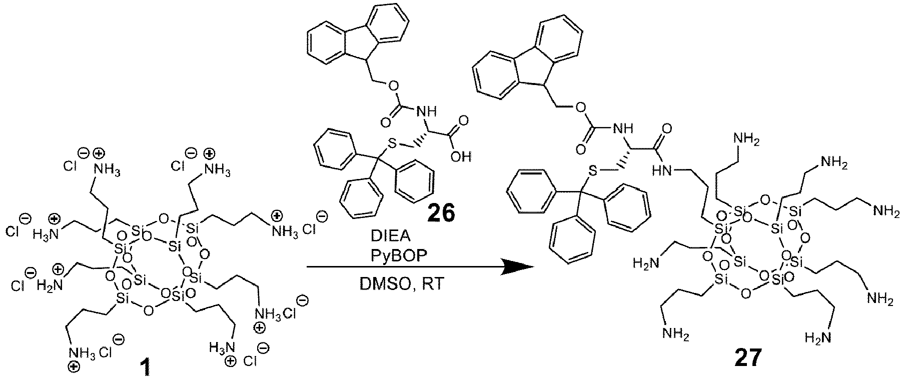

Eine Mischung aus 265,0 mg (0,45 mmol, 1 Äquivalent) Fmoc-Cys(Trt)-OH **26,** 1,89 mL (10,86 mmol, 24 Äquivalente) trockenem DIEA und 283,0 mg (0,54 mmol, 1,2 Äquivalente) PyBOP wurden in 10 mL trockenem DMSO gelöst und für 10 Minuten voraktiviert. 1,06 g (0,91 mmol, 2 Äquivalente) Octaammonium-POSS-Hydrochlorid 1 wurden in 10,0 mL trockenem DMSO gelöst und heftig gerührt. Die beschriebene Mischung wird mit einer peristaltischen Pumpe langsam hinzu gegeben. The Mischung wurde für weitere zwei Stunden bei Raumtemperatur gerührt. Danach wurde das Lösungsmittel durch Lyophilisation entfernt. Der Rückstand wurde in trockenem Acetonitril suspendiert und der unlösliche Teil wurde zweimal mit trockenem Acetonitril gewaschen. Der Niederschlag wurde in 10 Vol-% wässrigem MeCN mit 0,1 Vol-% TFA gelöst und mit einer semi-präparativen RP-HPLC gereinigt. Nach Lyophilisation wurden 145,0 mg eines weißen Feststoffes **27** erhalten (22,1%).
RP-HPLC, 10→50% B, t_{R}= 21,5 min.
ESI-MS Berechnung für C₆₁H₉₃N₉O₁₅SSi₈ m/z: 1449,19, gemessen 1449,7 [M+H]⁺.

### Synthese von Cys-Heptaguanidin-COSS (Cys-GuCOSS)

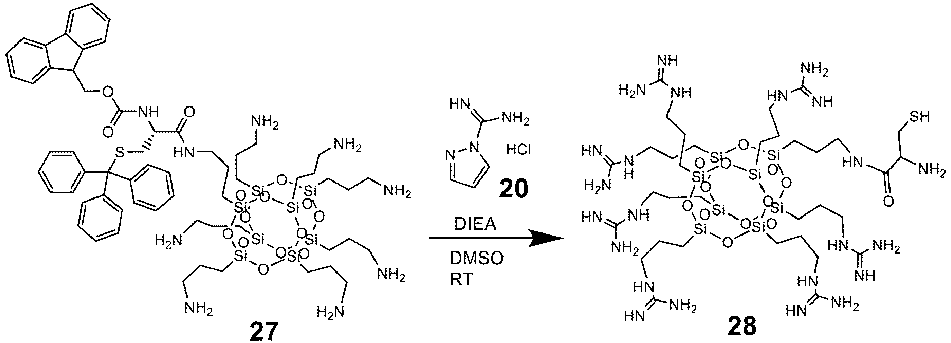

145 mg (0,12 mmol, 1 Äquivalent) Fmoc-Cys(Trt)-Heptaammonium-COSS **27**, 368 mg (2,49 mmol, 21 Äquivalente) 1H-Pyrazol-1-carboxamid-Hydrochlorid **20** und 823 µL (4,73 mmol, 40 Äquivalente) trockenes DIEA wurden in 10 mL trockenem DMSO gemischt und die Mischung wurde für 2 Tage bei Raumtemperatur gerührt. Danach wurde das Lösungsmittel durch Lyophilisation entfernt und der ölige Rückstand wurde in trockenem Acetonitril suspendiert. Der unlösliche Anteil wurde zweimal mit trockenem Acetonitril gewaschen. Der Niederschlag wurde in 10 Vol-% wässrigem MeCN mit 0,1 Vol-% TFA gelöst und mit einer semi-präparativen RP-HPLC gereinigt (Gradient 0 bis 50). Nach Lyophilisation wurde die Trityl-Schutzgruppe mit 1 mL 2 N HCl und 1 mL CHCl₃ entfernt und die Lösung wurde für 3 Stunden bei Raumtemperatur geschüttelt. Die organische Phase wurde entfernt und die wässrige Phase wurde zweimal mit je 1 mL CHCl₃ extrahiert. Die kombinierten organischen Phasen wurden verworfen und 9 mL deionisiertes Wasser wurde zu der wässrigen Phase gegeben. Das Lösungsmittel wurde durch Lyophilisation entfernt. Der verbleibende farblose Feststoff wurde in 5 mL 5 Vol-% wässriger AcOH gelöst und das Lösungsmittel wurde mittels Lyophilisation entfernt. 64,0 mg eines weißen Feststoffes **28** wurden erhalten (42,34%).
RP-HPLC, 0→50% B, t_{R}=20,12 min.
ESI-MS Berechnung für C₃₄H₈₃N₂₃O₁₃SSi₈ m/z: 640,46, gemessen 640,30 [M+H]⁺.

### Synthese von Aldrithiol-aktiviertem Doxorubicin 33

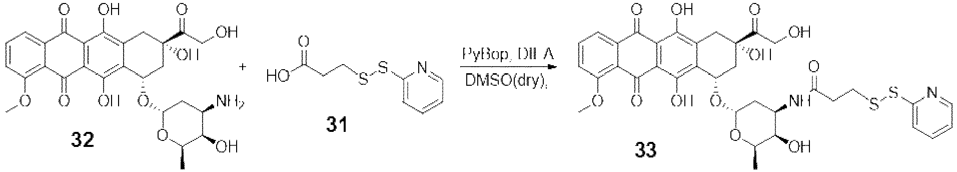

Aldrithiol-aktiviertes Doxorubicin **33** wurde aus 1 Äquivalent Doxorubicin **32** (10 mg, 0,018 mmol), 1,5 Äquivalenten Aldrithiol-aktivierter 3-Mercaptopropionsäure **31** (5,8 mg, 0,027 mmol), 1,2 Äquivalenten (11,2 mg, 0,22 mmol) PyBOP und 3 Äquivalenten DIEA (9,3 µL, 0,054 mmol) synthetisiert. Nach Ende der Reaktion (24 Stunden, Raumtemperatur) wurde die Reaktionsmischung lyophilisiert und mittels semi-präparativer RP-HPLC gereinigt.
RP-HPLC 25to100 % B: t_{R}: 11,95min
ESI-MS calc. for C₃₅H₃₆N₂O₁₂S₂ m/z: 740.80, meas. 741.29 [M+H]⁺.

### Synthese von GuCOSS-Doxorubicin-Konjugat 34

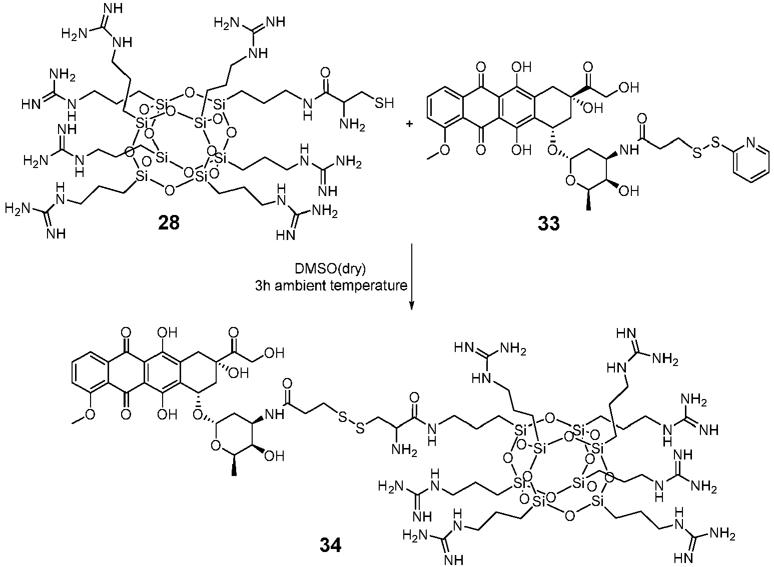

Das GuCOSS-Doxorubicin-Konjugat **34** wurde aus 1 Äquivalent Cystein-modifiziertem GuCOSS **28** (5 mg, 0,0039 mmol),in trockenem DMSO gelöst, und 1,2 Äquivalenten Aldrithiolaktivierte 3-Mercaptopropionsäure-Doxorubicin **33** (3,5 mg, 0,0047 mmol) synthetisiert. Nach Ende der Reaktion wurde die Reaktionsmischung lyophilisiert und mittels präparativer RP-HPLC gereinigt. Nach Lyophilisierung wurden 1.1mg (14.7%) des Zielproduktes **34** als rotes flockiges Pulver erhalten.
RP-HPLC 0to80% B: t_{R}: 14.63min
HR-MS calc. for C₆₄H₁₁₄N₂₄O₂₅S₂Si₈ m/z: 477.6568 meas. 477.6562 [M+4H]⁴⁺.

### Weitere Synthesen

Die Synthesen von 3-Carboxy-*N*,*N*,*N*-trimethylpropan-1-ammoniumchlorid **18** und 4-Trimethylammoniumbuttersäure-NHS-Ester **19** wurden gemäß Morano *et al.* durchgeführt (C. Morano, X. Zhang, L. D. Fricker, Anal Chem 2008, 80, 9298-9309).

### Laser-Scanning-Mikroskopie

Die zuvor synthetisierten fluoreszenzmarkierten Verbindungen 2 bis 7 (Verbindungen 2 und 5 sind Vergleichsbeispiele) wurden auf ihre Fähigkeit untersucht, in lebende Zellen einzudringen. Um die Aufnahme in die Zellen qualitativ abschätzen zu können, wurden lebende HeLa Zellen mittels Laser-Scanning-Mikroskopie untersucht.

Die Zellen wurden für 30 Minuten mit den Verbindungen 2 bis 7 bei einer Konzentration von 20 µM in serumfreiem Dulbecco's Modified Eagle Medium (DMEM) inkubiert. Danach wurden die Zellen dreimal mit PBS gewaschen und in DMEM mit fetalem Kälberserum analysiert. Die Analyse zeigte eine Aufnahme und intrazelluläre Anreicherung der Verbindungen, nicht nur im Zytoplasma, sondern auch im Nucleus und Nucleoli. Die Akkumulation der Verbindungen im Zellkern macht sie besonders interessant für die Verabreichung von Wirkstoffen, die in diesem Kompartiment wirken. Interessanterweise sammelten sich die Verbindungen mit größeren Kettenlängen der Reste verstärkt im Zytosol an.

Die Ergebnisse sind in FIG.1A und 1B gezeigt.

### Fluoreszenzmikroskopie

Mittels Fluoreszenzmikroskopie wurde untersucht, ob die Verbindungen in Zellen aller Domänen eindringen können. Es wurde gezeigt, dass die Verbindungen sowohl in Eukaryoten als auch in Prokaryoten eindringen können. Unter den untersuchten Zellen waren Hefe (*Saccharomyces cerevisiae*), Säugetierzellen (HeLa), Archaeen (*Sulfolobus islandicus, Sulfolobus tokodaii, Halobacterium salinarium*) und Bakterien (*E. coli*). Die Zellen wurden für 30 Minuten bei einer Konzentration von 20 µM mit den Verbindungen inkubiert. Das Durchdringen der Zellmembranen verschiedener Spezies und insbesondere von Archaeen, die eine außergewöhnliche Membranzusammensetzung haben, verdeutlichen, dass die Verbindungen vielseitige Transportverbindungen darstellen.

Die Ergebnisse für die HeLa-Zellen sind in FIG.1A und 1B gezeigt.

### Durchflusszytometrie

Um die Aufnahme der Verbindungen in eukaryotische Zellen zu quantifizieren, wurden umfangreiche Durchflusszytometrieuntersuchungen mit den Verbindungen 2 bis 7 (Verbindungen 2 und 5 sind Vergleichsbeispiele) durchgeführt. Ferner wurden auch die aus dem Stand der Technik bekannten zellpenetrierenden Peptide Tat **10,** Penetratin **11,** Heptaarginin **12** und Decaarginin **13** untersucht. Zu diesem Zweck wurden auch die Peptide mit TAMRA markiert.

Zunächst wurden HeLa-Zellen mit den zu untersuchenden Substanzen bei einer Konzentration von 20 µM in serumfreiem DMEM bei 37°C für 10 Minuten inkubiert. Danach wurden die Zellen dreimal mit PBS gewaschen und nach einem Trypsinverdau nochmals mit PBS gewaschen. Danach wurden die Untersuchungen durchgeführt. Substanz 4 zeigte die beste Aufnahme, die sogar 155mal stärker ausgeprägt war als die des Tat-Peptids. Die Ergebnisse sind in FIG.2 zusammen gefasst.

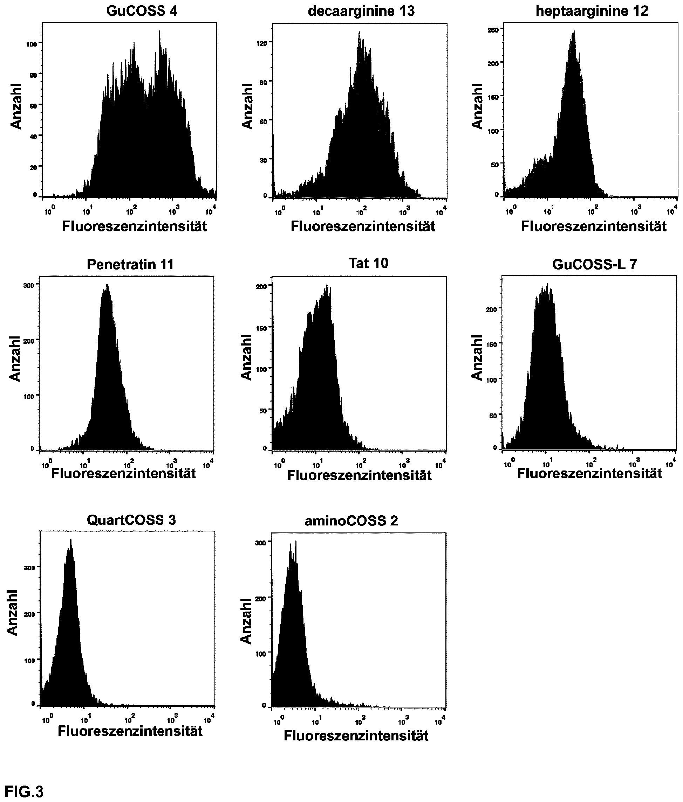

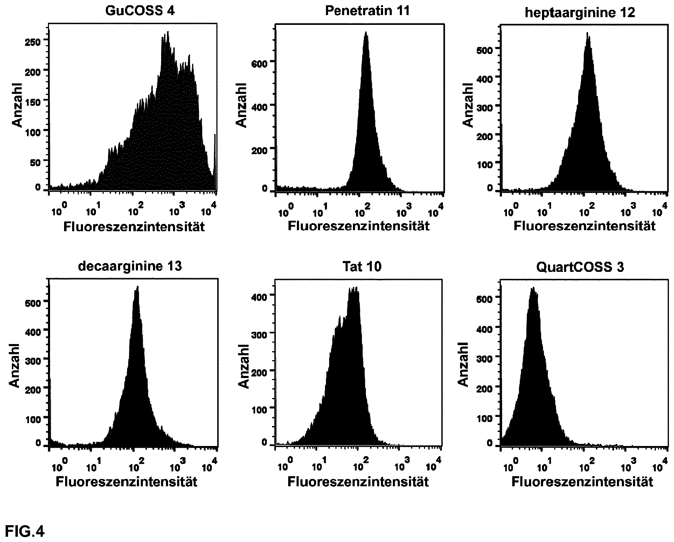

Ähnliche Ergebnisse wurden mit HEK-293- und CHO-Zellen erzielt. Die Ergebnisse sind in FIG.3 (HEK-293-Zellen) bzw. FIG.4 (CHO-Zellen) zusammen gefasst.

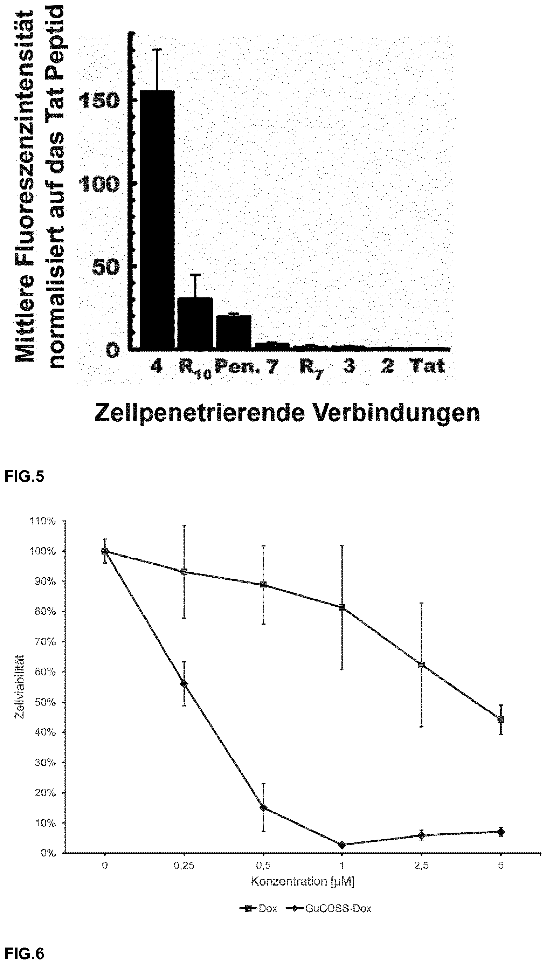

FIG.5 zeigt einen Vergleich der erzielten Fluoreszenzintensitäten für die verschiedenen zellpenetrierenden Verbindungen. Die Verbindungen 3, 4 und 7 sind erfindungsgemäße Strukturen.

### Untersuchung des Aufnahmemechanismus

Es wurden weitere Fluoreszenzuntersuchungen an Verbindung **4** durchgeführt. Dabei wurde wie oben beschrieben inkubiert, jedoch einmal bei 37°C und einmal bei 4°C. Überraschend wurde festgestellt, dass die Aufnahme der Verbindung **4** bei den unterschiedlichen Temperaturen kaum unterschiedlich war. Daraus kann angenommen werden, dass der Mechanismus energieunabhängig verläuft.

Ferner wurden HeLa-Zellen mit der Verbindung **4** bei unterschiedlichen Konzentrationen inkubiert. Schon bei 80 nM wurde eine Aufnahme in die Zellen beobachtet. Bei 1 µM wurde der größte Effekt beobachtet.

Darüber hinaus wurde untersucht, ob Serum die Aufnahme in die Zellen behindern kann. Dafür wurde die Inkubationslösung mit 2% fetalem Kälberserum versetzt. Es wurde kein Unterschied im Vergleich zu serumfreien Tests festgestellt.

### Wirkstofftransport in eine Zelle

Wie oben beschrieben wurde eine erfindungsgemäße Verbindung mit Doxorubicin als Cargo-Molekül synthetisiert. Um sicherzustellen, dass der Wirkstoff in der Zelle freigesetzt wird, wurde er mit einer Disulfidbindung an die Silsesquioxanstruktur gebunden.

Um die Toxizität von Doxorubicin auf HeLa-Zellen zu untersuchen, wurde ein Inkubationszeitabhängiger Test mit dem freien Wirkstoff durchgeführt. Dafür wurde Doxorubicin in PBS bei pH 5,5 gelöst und bei unterschiedlichen Konzentrationen für 1 Stunde, 2 Stunden und 3 Stunden mit 1x10⁴ HeLa-Zellen inkubiert. Nach dem Inkubieren wurden die Zellen mit DMEM gewaschen und nach 18 Stunden ein Zellproliferationstest (MTT) durchgeführt. Aufgrund der langsamen Aufnahme von Doxorubicin nach kurzer Inkubationszeit waren die Ergebnisse wie erwartet: mit längerer Inkubation nahm die Toxizität zu.

Danach wurde die oben gezeigte erfindungsgemäße Verbindung mit Doxorubicin als Cargo-Molekülstruktur getestet. Die Verbindung wurde - genau wie die Kontrollen (freies Doxorubicin, unbehandelte Zellen) -je dreifach bei gleicher Konzentration für eine Stunde inkubiert. Um die Disulfidbrücke zu schützen wurden die Zellen mit hoch-glucosehaltigem DMEM ohne Methionin, Cysteine und Glutamine gewaschen. Nach der Inkubation wurden die Zellen mit DMEM gewaschen und nach 18 Stunden wurde ein MTT-Assay durchgeführt. Die Ergebnisse zeigen klar, dass die Verbindung der Erfindung eine deutlich höhere Toxizität zeigte als freies Doxorubicin nach kurzer Inkubationszeit, was durch die schnelle Aufnahme der erfindungsgemäßen Verbindung in die Zelle erklärlich ist. Die Ergebnisse sind in FIG.6 zusammen gefasst.

## Patentansprüche

1. Silsesquioxanverbindungen mit einer der nachfolgend skizzierten chemischen Grundstrukturen T6, T8, T10 oder T12, 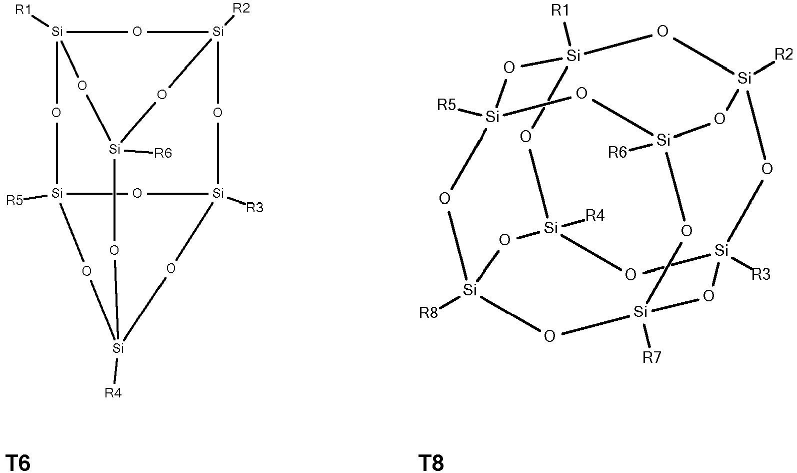 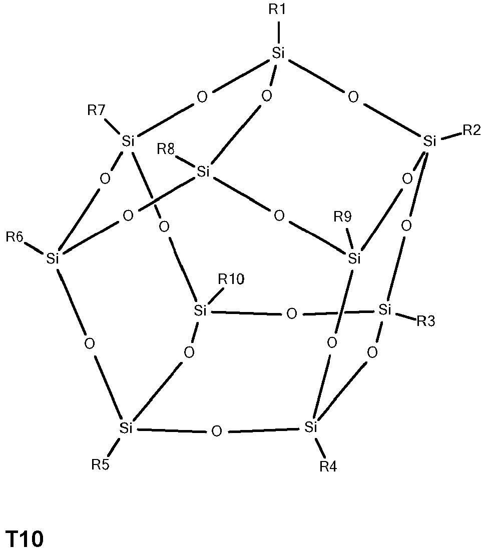 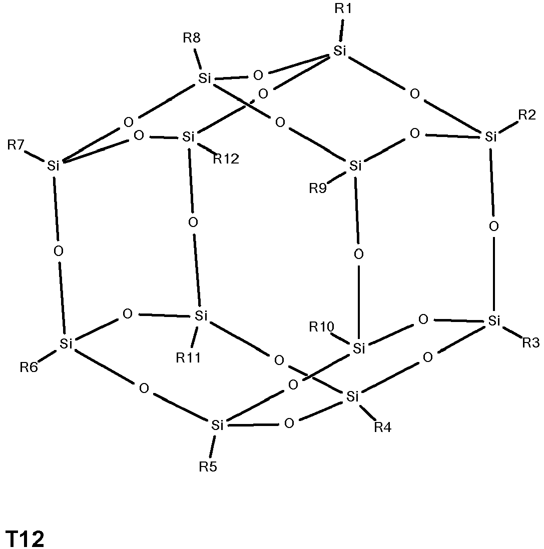 wobei wenigstens einer aus R1 bis R6 bei T6, R1 bis R8 bei T8, R1 bis R10 bei T10 oder R1 bis R12 bei T12 ein Rest ist, welcher eine oder mehrere Guanidingruppen und/oder quartäre Ammoniumgruppen aufweist,
wobei, wenigstens einer der Reste aus R1 bis R6 bei T6, R1 bis R8 bei T8, R1 bis R10 bei T10 oder R1 bis R12 bei T12, wenigstens eine Cargo-Molekülstruktur enthält, wobei die Cargo-Molekülstruktur ein Molekül enthält, das über Zellmembranen transportiert werden soll, und ausgewählt ist aus pharmazeutischen Wirkstoffen, Markerverbindungen, die innerhalb von Zellen ein messbares Signal erzeugen können, und Targeting-Strukturen, die geeignet sind, die Verbindungen an einem gewünschten Wirkort anzureichern, und wobei der Rest, der eine oder mehrere Guanidingruppen und/oder quartäre Ammoniumgruppen aufweist, aus der Strukturgruppe -X-A besteht,
- wobei A ausgewählt ist aus Guanidingruppe und quartärer Ammoniumgruppe, und
- wobei X substituiert oder unsubstituiert sein kann und ausgewählt ist aus verzweigtem oder unverzweigtem, zyklischem oder nicht zyklischem, gesättigtem oder ungesättigtem Alkylen und der Gruppe X₁-L-X₂,
• wobei X₁ und X₂ jeweils unabhängig voneinander substituiert oder unsubstituiert sein können und aus verzweigtem oder unverzweigtem, zyklischem oder nicht zyklischem, gesättigtem oder ungesättigtem Alkylen ausgewählt sind,
• wobei L in der Gruppe X₁-L-X₂ eine Linkergruppe ist, die ausgewählt ist aus
Carbonsäureamidgruppe, insbesondere Aminocarbonylgruppe (-HN-CO-) oder Carbonylaminogruppe (-CO-NH-); Carbonsäureestergruppe, insbesondere Oxycarbonyl (-O-CO-) oder Carbonyloxygruppe (-CO-O-), einschließlich der entsprechenden Thioester, Selenoester oder Phosphorester; Ethergruppe; Thioethergruppe; Imidgruppe; Imingruppe; Oxime; Sulfone einschließlich Sulfonamingruppen; Peroxidgruppen; Ketongruppen; Hydrazingruppen; Hydrazongruppen; Carbonsäurehydrazidgruppen und Disulfidbrücken.

2. Verbindung nach Anspruch 1, wobei X, X₁ und/oder X₂ gesättigtes, nicht-zyklisches C₁ bis C₁₀-Alkylen ist.

3. Verbindung nach wenigstens einem der vorhergehenden Ansprüche, wobei die quartäre Ammoniumgruppe eine Gruppe der Formel -N⁺RₓR_{y}R_{z} ist, wobei Rₓ, R_{y} und R_{z} unabhängig voneinander ausgewählt sind aus gesättigtem, nicht-zyklischem C₁- bis C₅-, insbesondere C₁- bis C₃-Alkyl.

4. Verbindung nach wenigstens einem der vorhergehenden Ansprüche, wobei L eine Carbonsäureamidgruppe ist und/oder X, X₁ und/oder X₂ C₃-Alkylen sind.

5. Verbindung nach wenigstens einem der vorhergehenden Ansprüche, wobei bei T6 wenigstens zwei der Reste R1 bis R6, bei T8 wenigstens vier der Reste R1 bis R8, bei T10 wenigstens sechs der Reste R1 bis R10 und bei T12 wenigstens acht der Reste R1 bis R12 eine oder mehrere Guanidingruppen und/oder quartäre Ammoniumgruppen aufweisen.

6. Verbindung nach wenigstens einem der vorhergehenden Ansprüche, wobei bei T6 wenigstens vier oder fünf der Reste R1 bis R6, bei T8 wenigstens sechs oder sieben der Reste R1 bis R8, bei T 10 wenigstens acht oder neun der Reste R1 bis R10 oder bei T12 wenigstens zehn oder elf der Reste R1 bis R12 eine oder mehrere Guanidingruppen und/oder quartäre Ammoniumgruppen aufweisen.

7. Verbindung nach wenigstens einem der vorhergehenden Ansprüche, wobei alle Reste R1 bis R6 bei T6, R1 bis R8 bei T8, R1 bis R10 bei T10 oder R1 bis R12 bei T12, die eine oder mehrere Guanidingruppen und/oder quartäre Ammoniumgruppen aufweisen, die gleiche chemische Konstitution haben.

8. Verbindung nach wenigstens einem der vorhergehenden Ansprüche, wobei diejenigen Reste R1 bis R6 bei T6, R1 bis R8 bei T8, R1 bis R10 bei T10 oder R1 bis R12 bei T12, die wenigstens eine Cargo-Molekülstruktur enthalten, eine Strukturgruppe Y-Z aufweisen oder aus der Strukturgruppe bestehen, wobei
Y substituiert oder unsubstituiert sein kann und aus verzweigtem oder unverzweigtem, zyklischem oder nicht zyklischem, gesättigtem oder ungesättigtem Alkylen ausgewählt ist, und Z die Cargo-Molekülstruktur enthält oder aus ihr besteht, wobei Z über eine funktionelle Gruppe direkt oder indirekt an Y gebunden ist, wobei die funktionelle Gruppe ausgewählt ist aus Carbonsäureamidgruppe, insbesondere Aminocarbonylgruppe (-HN-CO-) oder Carbonylaminogruppe (-CO-NH-); Carbonsäureestergruppe, insbesondere Oxycarbonyl (-O-CO-) oder Carbonyloxygruppe (-CO-O-), einschließlich der entsprechenden Thioester, Selenoester oder Phosphorester; Ethergruppe; Thioethergruppe; Imidgruppe; Imingruppe; Oxime; Sulfone einschließlich Sulfonamingruppen; Peroxidgruppen; Ketongruppen; Hydrazingruppen; Hydrazongruppen; Carbonsäurehydrazidgruppen und Disulfidbrücken.

9. Verbindung nach Anspruch 8, wobei Z folgende Cysteinstruktur aufweist, welche direkt oder indirekt an die Cargo-Molekülstruktur Q gebunden ist

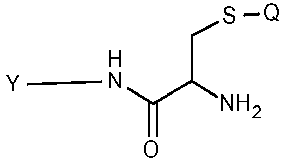

10. Verbindung nach einem der vorhergehenden Ansprüche zur Verwendung in einem therapeutischen oder diagnostischen Verfahren.

11. Verbindung nach einem der Ansprüche 1 bis 9 zur Verwendung in einem Verfahren, bei dem Moleküle über Zellmembranen transportiert werden.

12. Verfahren zur Herstellung einer Verbindung gemäß wenigstens einem der Ansprüche 1 bis 9, mit den Schritten:
a.) Bereitstellen eines Edukts E6, E8, E10 oder E12 mit nachfolgender Struktur: 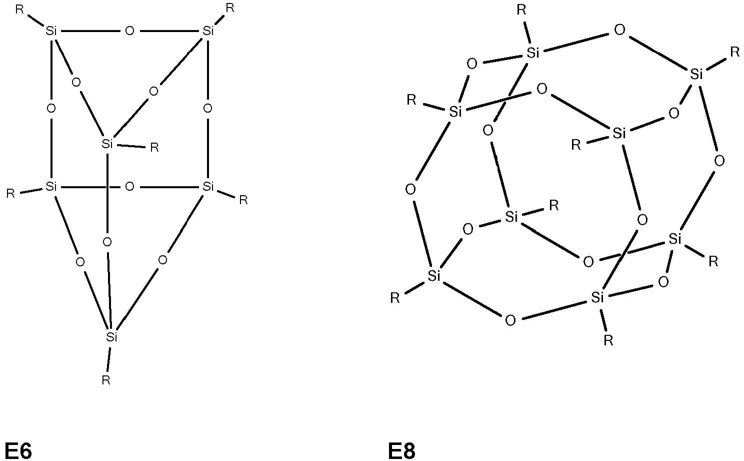 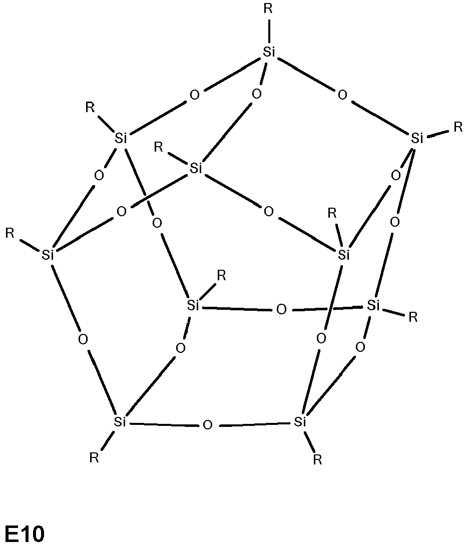 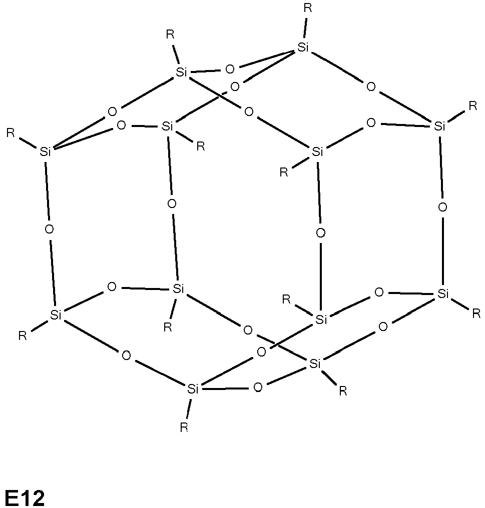 wobei wenigstens einer der Reste R aus der Strukturgruppe X-A* besteht, wobei A* eine protonierte oder nicht protonierte Aminogruppe ist, und wobei X substituiert oder unsubstituiert sein kann und ausgewählt ist aus verzweigtem oder unverzweigtem, zyklischem oder nicht zyklischem, gesättigtem oder ungesättigtem Alkylen,
b.) optional Binden einer oder mehrerer Cargo-Molekülstrukturen an einen oder mehrere Reste R, insbesondere durch Reaktion der Cargo-Molekülstruktur mit A*,
c1.) Umsetzen des Edukts mit einem Alkylierungsmittel, um durch Alkylierung von A* eine quartäre Ammoniumverbindung zu erhalten, oder
c2.) Umsetzen des Edukts mit einem Guanidinierungsmittel, um durch Guanidinierung von A* eine Guanidingruppe zu erhalten.

13. Verfahren nach Anspruch 12, wobei als Alkylierungsmittel Methyliodid und/oder als Guanidinierungsmittel 1H-Pyrazol-1-carboximidamid-hydrochlorid verwendet wird.

## Claims

1. Silsesquioxane compounds having one of the following basic chemical structures T6, T8, T10 or T12, 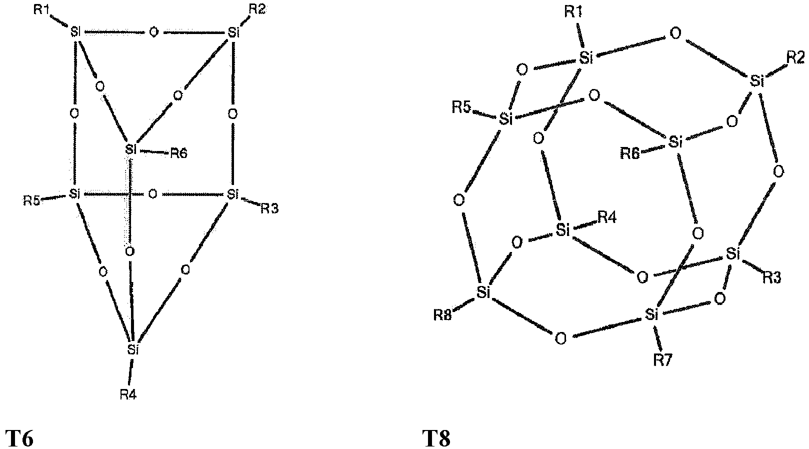 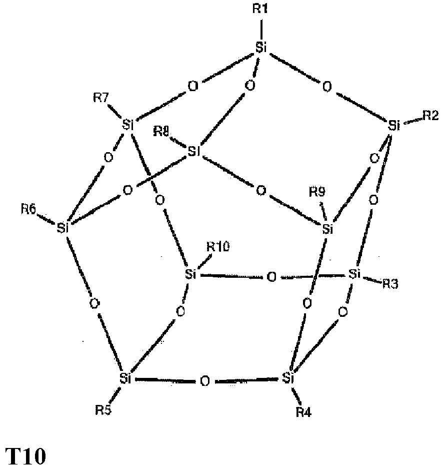 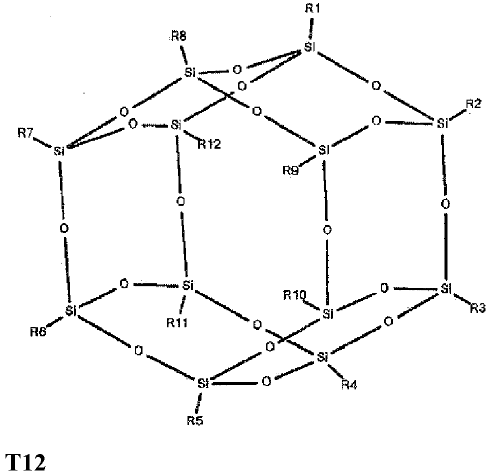 wherein at least one of R1 to R6 in T6, R1 to R8 in T8, R1 to RIO in T10 or R1 to R12 in T12 is a group which comprises one or more guanidine groups and/or quaternary ammonium groups,
wherein at least one of the groups of R1 to R6 in T6, R1 to R8 in T8, R1 to RIO in T10 or R1 to R12 in T12 contains at least one cargo molecular structure, wherein the cargo molecular structure contains a molecule which should be transported across cell membranes and is selected from active pharmaceutical ingredients, marker compounds which within cells are able to generate a measurable signal, and targeting structures which are suitable to accumulate the compounds at a desired site of action, and wherein the group which comprises one or more guanidine groups and/or quaternary ammonium groups consists of the structure group -X-A,
- wherein A is selected from guanidine group and quaternary ammonium group, and
- wherein X may be substituted or unsubstituted and is selected from branched or unbranched, cyclic or not cyclic, saturated or unsaturated alkylene and the group X₁-L-X₂,
• wherein X₁ and X₂ each independently from each other may be substituted or unsubstituted and are selected from branched or unbranched, cyclic or not cyclic, saturated or unsaturated alkylene,
• wherein L in group X₁-L-X₂ is a linker group which is selected from carboxylic acid amide group, in particular amino carbonyl group (-HN-CO-) or carbonyl amino group (-CO-NH-); carboxylic acid ester group, in particular oxycarbonyl (-O-CO-) or carbonyloxy group (-CO-O-), including the respective thioesters, selenoesters or phosphorous esters; ether group; thioether group; imide group; imine group; oximes; sulfones including sulfone amine groups; peroxide groups; ketone groups; hydrazine groups; hydrazone groups; carboxylic acid hydrazide groups and disulfide bridges.

2. Compound according to claim 1, wherein X, X₁ and/or X₂ is saturated, not cyclic C₁ to C₁₀ alkylene.

3. Compound according to at least one of the preceding claims, wherein the quaternary ammonium group is a group of the formula -N⁺RₓR_{y}R_{z}, wherein Rₓ, R_{y} and R_{z} independently from each other are selected from saturated, not cyclic C₁ to C₅, in particular C₁ to C₃ alkyl.

4. Compound according to at least one of the preceding claims, wherein L is a carboxylic acid amide group and/or X, X₁ and/or X₂ are C₃ alkylene.

5. Compound according to at least one of the preceding claims, wherein in T6 at least two of the groups R1 to R6, in T8 at least four of the groups R1 to R8, in T10 at least six of the groups R1 to RIO and in T12 at least eight of the groups R1 to R12 comprise one or more guanidine groups and/or quaternary ammonium groups.

6. Compound according to at least one of the preceding claims, wherein in T6 at least four or five of the groups R1 to R6, in T8 at least six or seven of the groups R1 to R8, in T10 at least eight or nine of the groups R1 to RIO or in T12 at least ten or eleven of the groups R1 to R12 comprise one or more guanidine groups and/or quaternary ammonium groups.

7. Compound according to at least one of the preceding claims, wherein all groups R1 to R6 in T6, R1 to R8 in T8, R1 to R10 in T10 or R1 to R12 in T12 which comprise one or more guanidine groups and/or quaternary ammonium groups have the same chemical constitution.

8. Compound according to at least one of the preceding claims, wherein those groups R1 to R6 in T6, R1 to R8 in T8, R1 to RIO in T10 or R1 to R12 in T12 which contain at least one cargo molecular structure comprise a structure group Y-Z or consist of said structure group, wherein
Y may be substituted or unsubstituted and is selected from branched or unbranched, cyclic or not cyclic, saturated or unsaturated alkylene, and
Z contains the cargo molecular structure or consists thereof, wherein Z is bonded directly or indirectly to Y via a functional group, wherein the functional group is selected from carboxylic acid amide group, in particular amino carbonyl group (-HN-CO-) or carbonyl amino group (-CO-NH-); carboxylic acid ester group, in particular oxycarbonyl (-O-CO-) or carbonyloxy group (-CO-O-), including the respective thioesters, selenoesters or phosphorous esters; ether group; thioether group; imide group; imine group; oximes; sulfones including sulfone amine groups; peroxide groups; ketone groups; hydrazine groups; hydrazone groups; carboxylic acid hydrazide groups and disulfide bridges.

9. Compound according to claim 8, wherein Z has the following cysteine structure which is bonded directly or indirectly to the cargo molecular structure

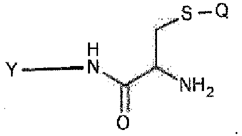

10. Compound according to one of the preceding claims for use in a therapeutic or diagnostic method.

11. Compound according to one of claims 1 to 9 for use in a method in which molecules are transported across cell membranes.

12. Method for the production of a compound according to at least one of claims 1 to 9, with the steps:
a.) providing of an educt E6, E8, E10 or E12 having the following structure: 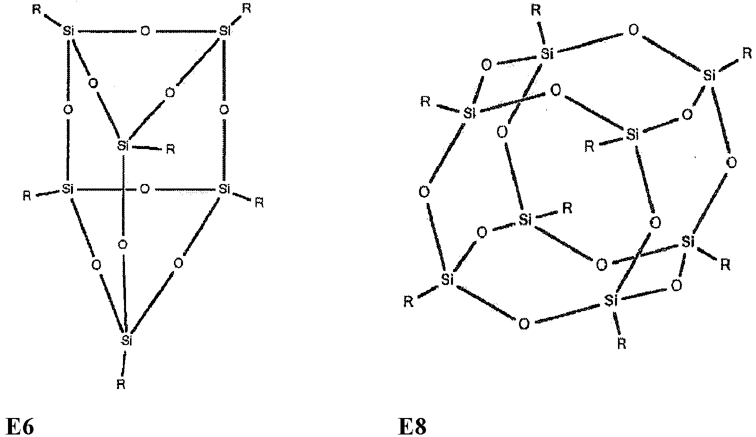 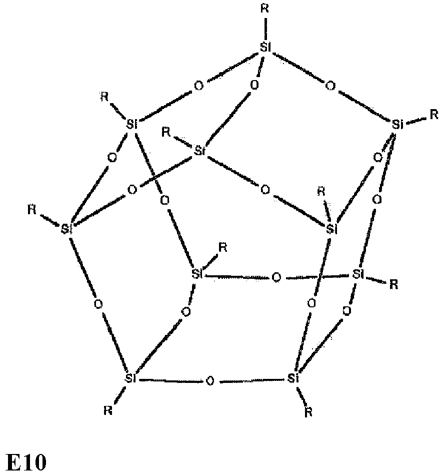 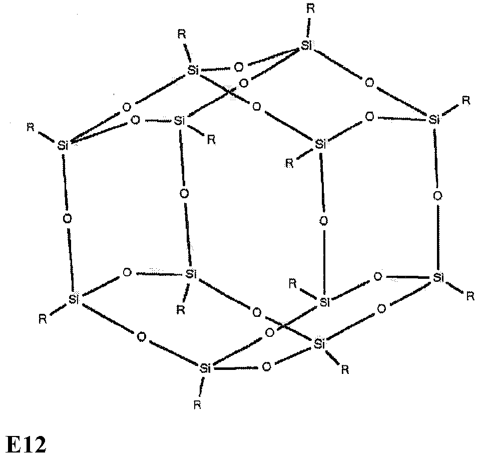 wherein at least one of the groups R consists of the structure group X-A*, wherein A* is a protonated or not protonated amino group, and wherein X may be substituted or unsubstituted and is selected from branched or unbranched, cyclic or not cyclic, saturated or unsaturated alkylene,
b.) optionally bonding of one or more cargo molecular structures to one or more groups R, in particular by reaction of the cargo molecular structure with A*,
c1.) converting the educt with an alkylating agent, for obtaining a quaternary ammonium compound by alkylation of A*, or
c2.) converting the educt with a guanidinating agent, for obtaining a guanidine group by guanidination of A*.

13. Method according to claim 12, wherein as alkylating agent methyl iodide and/or as guanidinating agent 1H-pyrazole-1-carboximide amide hydrochloride is used.

## Revendications

1. Composés de silsesquioxane comportant l'une des structures chimiques de base T6, T8, T10 ou T12 esquissées ci-dessous, 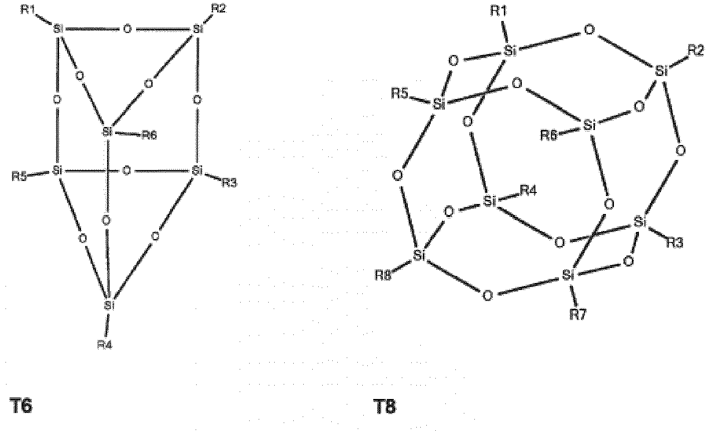 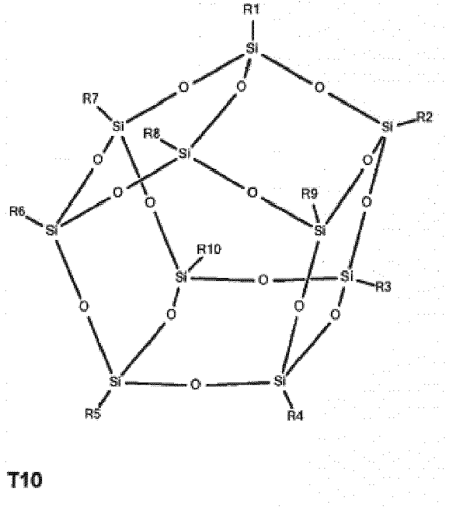 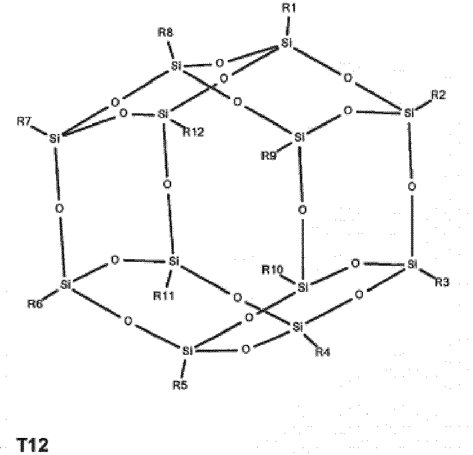 dans lequel l'un au moins parmi R1 à R6 en T6, R1 à R8 en T8, R1 à R10 en T10 ou R1 à R12 en T12 est un radical qui comporte un ou plusieurs groupes guanidine et/ou groupes ammonium quaternaire,
dans lequel l'un au moins des radicaux de R1 à R6 en T6, R1 à R8 en T8, R1 à R10 en T10 ou R1 à R12 en T12, contient au moins une structure moléculaire cargo, ladite structure moléculaire cargo contenant une molécule qui doit être transportée sur les membranes cellulaires et qui est sélectionnée parmi les principes pharmaceutiquement actifs, les composés marqueurs qui peuvent générer un signal mesurable dans les cellules et les structures de ciblage qui sont appropriées pour concentrer les composés sur un site d'action souhaité, et
dans lequel le radical, qui comporte un ou plusieurs groupes guanidine et/ou groupes ammonium quaternaire, comprend le groupe structurel -X-A,
- dans lequel A est choisi parmi le groupe guanidine et le groupe ammonium quaternaire, et
- dans lequel X peut être substitué ou non et est choisi parmi les alkylènes ramifiés ou non ramifiés, cycliques ou non cycliques, saturés ou insaturés et le groupe X₁-L-X₂,
• dans lequel X₁ et X₂ peuvent être substitués ou non substitués chacun indépendamment l'un de l'autre et sont sélectionné parmi les alkylènes ramifiés ou non ramifiés, cycliques ou non cycliques, saturés ou insaturés,
• dans lequel L du groupe X₁-L-X₂ étant un groupe de liaison qui est choisi parmi le groupe carboxamide, en particulier le groupe aminocarbonyle (-HN-CO-) ou le groupe carbonylamino (-CO-NH-) ; un groupe ester d'acide carboxylique, en particulier un groupe oxycarbonyl (-O-CO-) ou carbonyloxy (-CO-O-), y compris les thioesters, sélénoesters ou esters phosphoriques correspondants ; un groupe éther ; un groupe thioéther ; un groupe imide ; un groupe imine ; les oximes ; les sulfones, y compris les groupes sulfonamine ; les groupes peroxyde ; les groupes cétoniques ; les groupes hydrazine ; les groupes hydrazone ; les groupes hydrazide d'acide carboxylique et les ponts disulfure.

2. Composé selon la revendication 1, dans lequel X, X₁ et/ou X₂ sont un alkylène en C₁ à C₁₀ saturé non cyclique.

3. Composé selon l'une au moins des revendications précédentes, dans lequel le groupe ammonium quaternaire est un groupe de la formule -N⁺RₓR_{y}R_{z}, dans lequel Rₓ, R_{y} et R_{z} sont sélectionnés indépendamment parmi les alkyles saturés et non cycliques, en C₁ à C₅, en particulier en C₁ à C₃.

4. Composé selon l'une au moins des revendications précédentes, dans lequel L est un groupe carboxamide et/ou X, X₁ et/ou X₂ étant un alkylène en C₃.

5. Composé selon l'une au moins des revendications précédentes, dans lequel au moins deux des radicaux R1 à R6 en T6, au moins quatre des radicaux R1 à R8 en T8, au moins six des radicaux R1 à R10 en T10 et au moins huit des radicaux R1 à R12 en T12 comportent un ou plusieurs groupes guanidine et/ou groupes ammonium quaternaire.

6. Composé selon l'une au moins des revendications précédentes, dans lequel au moins quatre ou cinq des radicaux R1 à R6 en T6, au moins six ou sept des radicaux R1 à R8 en T8, au moins huit ou neuf des radicaux R1 à R10 en T10 ou au moins dix ou onze des radicaux R1 à R12 en T12 comportent un ou plusieurs groupes guanidine et/ou groupes ammonium quaternaire.

7. Composé selon l'une au moins des revendications précédentes, dans lequel tous les radicaux R1 à R6 en T6, R1 à R8 en T8, R1 à R10 en T10 ou R1 à R12 en T12, qui comportent un ou plusieurs groupes guanidine et/ou groupes ammonium quaternaire, ont la même constitution chimique.

8. Composé selon l'une au moins des revendications précédentes, dans lequel les radicaux R1 à R6 en T6, R1 à R8 en T8, R1 à R10 en T10 ou R1 à R12 en T12, qui contiennent au moins une structure moléculaire cargo, comportent un groupe structurel Y-Z ou forment le groupe structurel, dans lequel
Y peut être substitué ou non-substitué et est choisi parmi les alkylènes ramifiés ou non ramifiés, cycliques ou non cycliques, saturés ou insaturés, et
Z contient la structure moléculaire cargo ou est celle-ci, Z étant lié directement ou indirectement à Y par un groupe fonctionnel, le groupe fonctionnel étant sélectionné parmi le groupe carboxamide, en particulier le groupe aminocarbonyle (-HN-CO-) ou le groupe carbonylamino (-CO-NH-) ; un groupe ester d'acide carboxylique, en particulier un groupe oxycarbonyl (-O-CO-) ou carbonyloxy (-CO-O-), y compris les thioesters, sélénoesters ou esters phosphoriques correspondants ; un groupe éther ; un groupe thioéther ; un groupe imide ; un groupe imine ; les oximes ; les sulfones, y compris les groupes sulfonamine ; les groupes peroxyde ; les groupes cétoniques ; les groupes hydrazine ; les groupes hydrazone ; les groupes hydrazide d'acide carboxylique et les ponts disulfure.

9. Composé selon la revendication 8, dans lequel Z comporte la structure cystéine suivante qui est liée directement ou indirectement à la structure moléculaire cargo Q

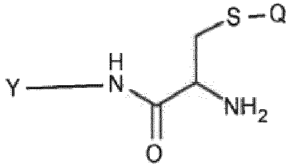

10. Composé selon l'une des revendications précédentes destiné à être utilisé dans un procédé thérapeutique ou diagnostique.

11. Composé selon l'une des revendications 1 à 9 destiné à être utilisé dans un procédé dans lequel des molécules sont transportées sur à travers des membranes cellulaires.

12. Procédé de production d'un composé selon l'une au moins des revendications 1 à 9, le procédé comprenant les étapes suivantes :
a.) fournir un réactif E6, E8, E10 ou E12 ayant la structure suivante : 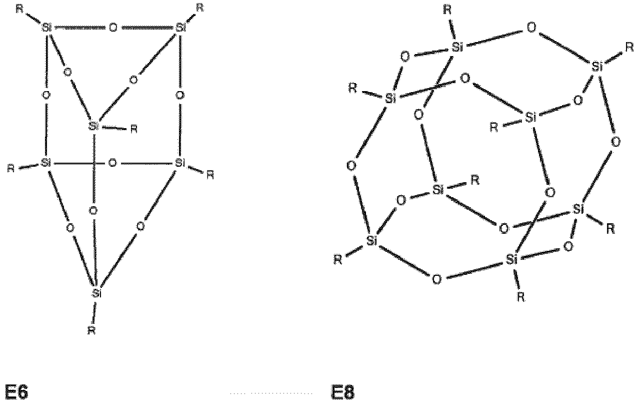 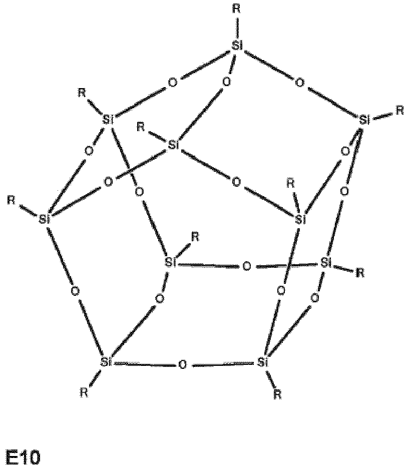 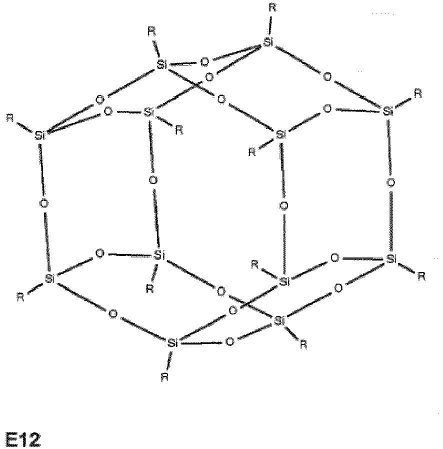 dans lequel l'un au moins des radicaux R est le groupe structural X-A*, dans lequel A* est un groupe amino protoné ou non protoné, et dans lequel X peut être substitué ou non substitué et esty choisi parmi les alkylènes ramifiés ou non ramifiés, cycliques ou non cycliques, saturés ou insaturés,
b.) éventuellement lier une ou plusieurs structures moléculaires cargo à un ou plusieurs radicaux R, en particulier par réaction de la structure moléculaire cargo avec A*,
c1.) faire réagir le réactif avec un agent d'alkylation pour obtenir un composé ammonium quaternaire par alkylation de A*, ou
c2.) faire réagir le réactif avec un agent de guanidination pour obtenir un groupe guanidine par guanidination de A*.

13. Procédé selon la revendication 12, dans lequel de l'iodure de méthyle est utilisé comme agent d'alkylation et/ou du chlorhydrate de 1H-pyrazole-1-carboximidamide est utilisé comme agent de guanidination.
